Europäisches Patentamt

European Patent Office

Office européen des brevets

(11) Publication number: **0 103 409 B1**

# EUROPEAN PATENT SPECIFICATION

(45) Date of publication of patent specification: **10.02.93**

(51) Int. Cl.5: **C12N 15/15**, C12P 21/02, C12N 1/19, C12N 15/81, A61K 37/64, //C12R1/865

(21) Application number: **83304668.3**

(22) Date of filing: **12.08.83**

Divisional application 88201179 filed on 08.06.88.

(54) **Glycolytic promoters for regulated protein expression: protease inhibitor.**

(30) Priority: **13.08.82 US 408099**
**28.04.83 US 489406**

(43) Date of publication of application:
**21.03.84 Bulletin 84/12**

(45) Publication of the grant of the patent:
**10.02.93 Bulletin 93/06**

(84) Designated Contracting States:
**BE CH DE FR GB IT LI NL SE**

(56) References cited:
EP-A- 0 114 777
EP-A- 0 120 551
EP-A- 0 137 633
BE-A- 895 961

JOURNAL OF MOLECULAR AND APPLIED GE-
NETICS, vol. 1, no. 5, 1982, pp. 419-434;
Raven Press, New York, US T. ALBER et al.:
"Nucleotide sequence of the triose phos-
phate isomerase gene of Saccharomyces
cerevisiae"

(73) Proprietor: **ZYMOGENETICS, INC.**
**2121 North 35th Street**
**Seattle Washington 98103(US)**

(72) Inventor: **Kawasaki, Glenn Hitoshi**
**1547 16th East**
**Seattle Washington 98112(US)**
Inventor: **Woodbury, Richard Grant**
**15464 10th Avenue, N.E.**
**Seattle Washington 98155(US)**

(74) Representative: **Allard, Susan Joyce et al**
**BOULT, WADE & TENNANT, 27 Furnival**
**Street**
**London EC4A 1PO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

BIOCHEMISTRY, vol. 21, no. 8, April 1982, pp. 1935-1942, American Chemical Society, US D. CLIFTON et al.: "Mutant studies of yeast fructokinase." P. 1941

FEBS LETTERS, vol. 130, no. 2, August 1981, pp. 297-300; Elsevier/North-Holland Biomedical Press, NL - J. CARLSON et al.: "Rat alpha1-antitrypsin. Preliminary characterisation of the in vitro mRNA translation product."

BIOCHEMICAL AND BIOPHYSICAL RESEARCH COMMUNICATIONS, vol. 103, no. 2, November 30, 1981, pp. 751-758; Academic Press Inc. - T. CHANDRA et al.: "Induction of alpha1-antitrypsin mRNA and cloning of its cDNA." PP. 755-757

PROCEEDINGS OF THE NATIONAL ACADEMY OF SCIENCES USA, vol. 78, no. 11, November 1981, pp. 6826-6830 - K. KURACHI et al.: "Cloning and sequence of cDNA coding for alpha1-antitrypsin."

NATURE, vol. 297, June 24, 1981, pp. 655-659; Macmillan Journals Ltd. - M. LEICHT et al.: "Sequence homology and structural comparison between the chromosomal human alpha1-antitrypsin and chicken ovalbumin genes"

NATURE, vol. 298, July 22, 1982, no. 5872, pp. 347-350; Macmillan Journals Ltd, Chesham, Bucks, GB - P. Valenzuela et al.: "Synthesis and assembly of hepatitis B virus surface antigen particles in yeast."

BIOCHEMICAL AND BIOPHYSICAL RESEARCH COMMUNICATIONS, vol. 108, no. 3, October 15, 1982, pp. 1107-1112; Academic Press Inc., US - G. KAWASAKI et al.: "Cloning of yeast glycolysis genes by complementation."

**Description**

The ability to obtain expression of foreign, i.e., exogenous, DNA in unicellular microorganisms provided the opportunity to conveniently prepare long polypeptide chains of interest. Almost immediately, varied polypeptides, such as the small hormone somatostatin and more sophisticated polypeptides, such as insulin, interferons, thymosin and a variety of vaccines having capsid proteins, were prepared and reported in the literature. For the most part, the initial work was performed in the bacterium E. coli which had been the subject of intensive study because scientists were familiar with many aspects of its genetic structure and properties. Initial attention was therefore directed to producing foreign proteins in E. coli. Once the ability to employ E. coli as a host was established, the limitations and disadvantages of employing E. coli encouraged the use of other hosts.

One host which appeared to be particularly attractive because it lacked many of the shortcomings of E. coli was yeast. However, yeast is a eukaryote and, therefore, has a more sophisticated genetic system. Furthermore, less is known about the yeast genome than is known about E. coli. In order to use yeast as a host for the production of proteins foreign to yeast, a number of discoveries are required, and new materials must be made available.

Initially, a replication system was required which provided stability in yeast, either as an extrachromosomal element or by integration into the yeast chromosome. In addition, the regulatory functions concerned with transcription and expression had to be developed in order to allow for expression of the desired protein. There was also the uncertainty whether foreign DNA sequences would be transcribed and translated and, if expressed, whether the resulting polypeptides would survive in the yeast cell. Also remaining to be determined was the effect of the foreign proteins on the viability of the yeast cell, such as the effect of recombinant DNA (RDNA) on mitosis, sporulation and vegetative growth.

There have, therefore, been substantial efforts to develop novel RDNA systems in yeast, which will allow for regulated expression of a protein of interest, as well as highly efficient production of such proteins.

Hitzeman et al., J. Biol. Chem., 255:12073-12080 (1980) describe a plasmid having a yeast 3-phosphoglycerate kinase (PGK) gene and accompanying regulatory signals capable of expression in yeast. Other references of interest include Clifton, et al., Genetics, 88:1-11 (1978); Clark and Carbon, Cell, 9:91-99 (1976); Thomson, Gene, 1:347-356 (1977); Holland and Holland, J.Biol. Chem., 254:5466-5474 (1979); Holland and Holland, ibid. 254:9830-9845 (1979); Nasmyth and Reed, Proc. Nat. Acad. Sci., 77:2119-2123 (1980); Broach, et al., Gene, 8:121-133 (1979); and Williamson, et al., Nature, 283: 214-216 (1980).

EP-A-073657 discloses an extrachromosomal element which is capable of replication in yeast and which contains a yeast promoter capable of regulating the transcription of a glycolytic protein, the promoter being followed downstream by a gene expressing a protein other than that normally regulated by such a promoter.

Chandra et al, Biochem. [Biophys.] Res. Comm. 103(2), (1981) pp.751-758 disclose a substantially unglycosylated, baboon pre-pro alpha-1-antitrypsin.

Alber and Kawasaki, J. Molecular and Applied Genetics, Vol 1 (5) (1982) p 419-434 disclose the sequence of the triose phosphate isomerase gene and promoter.

Kurachi et al, Proc. Natl. Acad. Sci. USA Vol 78 (11) (1981) p 6826-6836 disclose the sequence of human alpha-1-antitrypsin.

In the accompanying FIGURES:

FIGS. 1A and 1B are cDNA sequences of two forms of genes coding human alpha-1-antitrypsin.

FIG. 2 illustrates the restriction maps of plasmids CTEA32 and CAT1.

FIG. 3 is a diagram of the electrophoresis chromatogram showing purified alpha-1-antitrypsin produced according to the present invention.

FIG. 4 illustrates the restriction map of plasmid C1/1.

FIG. 5 illustrates the DNA sequence of the multiple restriction site of pUC13.

FIG. 6 illustrates the restriction map of plasmid pUCα1 containing the DNA sequence from FIG. 1.

FIG. 7 is the restriction map of plasmid HAT4.

Novel yeast promoters are provided which control the transcription of genes in the glycolytic pathway and which find use in the regulated production of proteins foreign to the yeast. Promoters of particular interest include the promoters for triose phosphate isomerase, pyruvate kinase, phosphoglucose isomerase, phosphoglycerate mutase, hexokinase 1, hexokinase 2, glucokinase, phosphofructo kinase, and aldolase, as well as the glycolytic regulatory gene. The protease inhibitor, mammalian alpha-1-antitrypsin, is expressed using the promoter for triose phosphate isomerase.

Methods and compositions are provided for regulated efficient expression of alien or foreign DNA in a yeast host. (Alien or foreign DNA is DNA not naturally occurring in the wild type particularly from a different

EP 0 103 409 B1

species and which does not normally exchange genetic information with the host.) Novel promoters are employed which are involved in the glycolytic pathway and provide for high levels of protein production, so that a substantial proportion of the total protein produced by the yeast cells can be dedicated to the protein of interest. In addition, regulatory mechanisms associated with regulation of production of the glycolytic enzymes are achieved, so that production of the desired products may be modulated. Furthermore, viable cells can be maintained to enhance the efficiency and amount of expression.

The promoters of interest are those promoters involved with expression of triose phosphate isomerase and pyruvate kinase, which are controlled by the glycolytic regulation gene GCR1. The genes of the glycolytic pathway include hexokinase 1 and 2 (HXK1,2); phosphoglucose isomerase (PGI), triose phosphate isomerase (TPI); phosphoglycerate kinase (PGK), phosphoglycerate mutase (GPM), pyruvate kinase (PYK), phosphofructo kinase (PFK), enolase (ENO); fructose 1,6-diphosphate aldolase (FDA); glyceraldehyde 3-phosphate dehydrogenase (GLD); and glycolysis regulation protein (GCR).

The promoters may be obtained by employing a gene bank having large fragments of yeast DNA. By introducing the fragments into appropriate vectors, particularly shuttle vectors having replicons for prokaryotes and yeast, one can readily amplify and clone the yeast DNA In a bacterium and then introduce the yeast DNA into mutant yeast cells for complementation. In this manner, yeast fragments can be identified which complement auxotrophic lesions or mutations in a yeast host.

Of particular interest, is where the host is auxotrophic in both the glycolytic pathway step of interest and a separate biochemical pathway, which is complemented by a marker in the vector. Once having established a DNA segment having the desired gene, one may reclone by various techniques to shorten the DNA segment and provide for a segment which is primarily the gene of interest in conjunction with its regulatory signals for transcription and expression.

In order to retain the promoter, it is essential that the initiator methionine be determined and this codon be used for developing the strategy for introducing the alien DNA downstream from the promoter. Various techniques can be employed for providing a site for introduction of the alien DNA so as to be under the regulatory control of the promoter in the glycolytic pathway.

Where a restriction site is conveniently adjacent to the initiator methionine codon, the glycolytic gene may be cleaved at that site and the DNA chewed back with Bal31 for varying periods of time, so as to chew into or past the initiator methionine codon or retain the initiator methionine codon.

Where there is no convenient restriction site, other splicing techniques such as primer repair may be employed. Also, by employing in vitro mutagenesis, one can introduce a restriction site adjacent the initiator methionine, which encodes for the initial amino acids of the desired protein. In each instance, a linearized DNA segment is obtained having the intact promoter for the glycolytic product and normally includes other DNA sequences, such as an intact replicon, one or more markers, and the like.

Exemplary of the above procedure is the development of a vector having the promoter for the TPI1 gene. An exemplary vector CV13 having the replicons or replication systems from pBR322 and 2μ-plasmid of yeast, as well as the LEU2 gene was employed for insertion of a yeast fragment which was shown to have the TPI1 gene. This was achieved by employing double selection with a mutant yeast which was leu⁻, tpi⁻. The TPI1 gene was found to have a unique KpnI site. The vector was cleaved at the KpnI site and then treated with the double stranded exonuclease Bal31 for varying times to chew back the DNA to about the f-met codon. Linkers were then inserted providing desired restriction sites. Alien DNA could then be inserted providing a sequence having a f-met codon in the appropriate position for initiation. Alternatively, the foreign DNA can be expressed using the f-met codon of the TPI1 gene.

Similar procedures can be performed with the other subject glycolytic genes in order to provide the promoters associated with those genes. The PYK sequence has a convenient XbaI site for restriction, where the few additional bases may be removed, if required, using Bal31 for a short period of time to chew to or through the methionine codon. Of particular interest is the use of the GCR promoter to control the expression of the other genes involved in the glycolytic pathway. By employing the GCR gene, in conjunction with other glycolytic promoters regulating expression of alien DNA, one can turn on and off the other promoters, so as to regulate the expression of the alien DNA. Thus, one can allow vegetative growth to proceed until a desired cell density is achieved, before permitting production of the desired polypeptide.

By employing appropriate auxotrophs, one can further regulate the expression of the polypeptides of interest in choosing the appropriate nutrient medium. Where the chosen promoter is repressed by the particular nutrient because of a metabolic block, a change in the nature of the nutrient can induce expression. Furthermore, the activity of a number of promoters in the glycolytic pathway can be affected by the repression or activation of expression by the GCR gene or other regulatory controls. Also, the GCR regulatory signals can be used to titrate the polypeptide functioning as the regulator for expression of GCR. By having vectors whose copy number can be controlled, one can vary the activity of the wild type GCR

4

gene.

In order to obtain expression, an extrachromosomal element construct will be prepared having a number of sequences defining different functions. One function is the replication system, which forms part of a vector. Another function is a promoter by itself or in conjunction with the alien DNA. Other functions include initiators and terminators of expression. Also, there will be selectable markers.

In developing an appropriate vector, while not necessary, it will be common to have both a replication system for yeast and a replication system for a prokaryote (a shuttle vector). The replication system for yeast may be one which provides for stable maintenance of an extrachromosomal element or one which provides a sufficient lifetime for the DNA in the host, that there is an acceptable probability of integration of the DNA into the host. Integration can be greatly aided by providing for a sequence homologous to the host DNA, so as to provide for recombination. Generally, the homologous sequence will be at least about 800bp usually not more than about 2000bp. Therefore, either integration or an autonomous replication system, such as the use of the ARS1 gene, may be employed to provide for the maintenance of the alien DNA in the yeast host. The replication system which is chosen should provide for a reasonable copy number usually greater than 1, preferably greater than 5. A wide variety of replication systems are available on a wide variety of prokaryotic vectors, such as pBR322, pACYC184, pSC101, pMB9, etc. Alternatively, one or more copies of the DNA construct can be integrated into the host chromosome. The replication systems may also be conditionally regulated, usually being temperature sensitive so that replication can be turned on and off by varying the temperature.

In addition to the replication system, there will also be one or more selectable markers, there usually being at least one marker in addition to the alien DNA, which may serve as a marker. Conventional markers include biocidal markers providing antibiotic resistance and those providing resistance to toxins and heavy metal. Also useful is employing an auxotrophic host and providing prototrophy by complementation. In addition to the conventional selection systems just described, the glycolytic genes of the present invention are particularly desirable markers since they can provide for selection, using sugars as selective substrates, in appropriate mutant host strains.

Other genes may also be inserted into the extrachromosomal element for a variety of purposes. Where integration is desirable in the genome of the host, a homologous sequence for a particular region of the host genome may be included in the extrachromosomal element. Where amplification of one or more sequences is desired, genes known to provide such amplification, such as dihydrofolate reductase genes, which respond to methotrexate stress or metallothionein genes, which respond to heavy metal stress, may be included in the extrachromosomal element, flanked by the DNA regions to be reiterated. Other regulatory signals may also be included, such as centromeres, autonomously replicating segments, etc.

In order to isolate the promoters of interest, clones can be made of yeast chromosomal DNA by random digestion or mechanical shearing of the yeast genome. The presence of the desired gene is then determined by introducing a homogeneous clone of a yeast fragment into an auxotrophic host for complementation. Desirably, the cloning vehicle may have another gene which allows for an additional basis for selection, so that double selection techniques can be used. The mutants are substantially incapable of growing on limited nutrient medium, so that one can select for the presence of the desired glycolytic gene by the choice of medium. After isolating the yeast fragment having the desired gene, the fragment may be subcloned so as to remove superfluous DNA flanking regions and provide for a fragment which is more easily manipulated. The smaller fragment containing the desired gene, of a size less than about 500 base pairs may then be further cloned, restriction mapped and sequenced, so as to provide a useful source for the desired promoters and insertion of the alien DNA. Also, as indicated, the promoters in themselves may be useful, in acting as a titrater for repressor or activator, where it is desirable to modulate the production of a particular enzyme in the yeast host. The alien DNA may be from any source, either naturally occurring or synthetic, either prokaryotic or eukaryotic. Of particular interest are mammalian genes which express a poly(amino acid), that is, polypeptide or protein which has physiological activity. To varying degrees, poly-(amino acids) prepared in yeast may be modified by glycosylation, where the glycosylation may not occur or may occur at different sites from the naturally occurring mammalian polypeptide and/or in different degrees with different saccharides. It is therefore of great interest to be able to prepare polypeptides which are different from the naturally occurring polypeptide by the degree and manner of glycosylation and in many instances may differ in one or more ways as to the amino acid sequences, where there may be deletions of one or more amino acids or substitutions of one or more amino acids. Mammalian genes may come from a wide variety of mammalian sources, such as domestic animals (e.g. bovine, porcine, ovine and equine) and primates e.g. humans and monkeys.

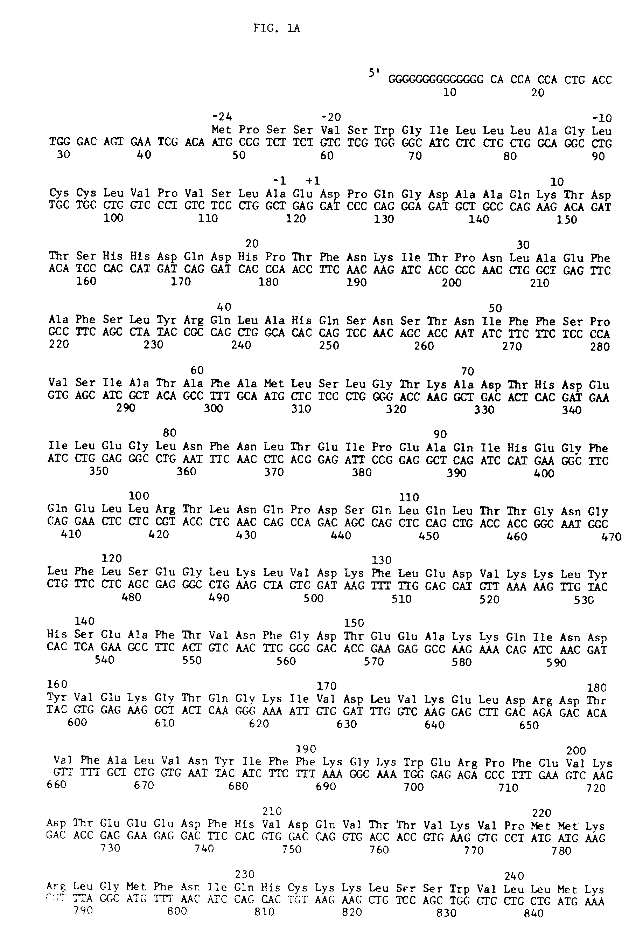

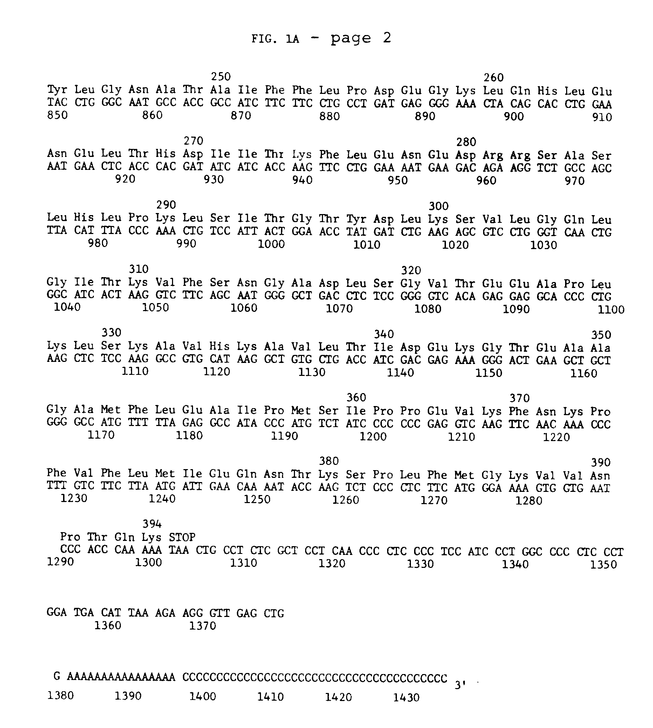

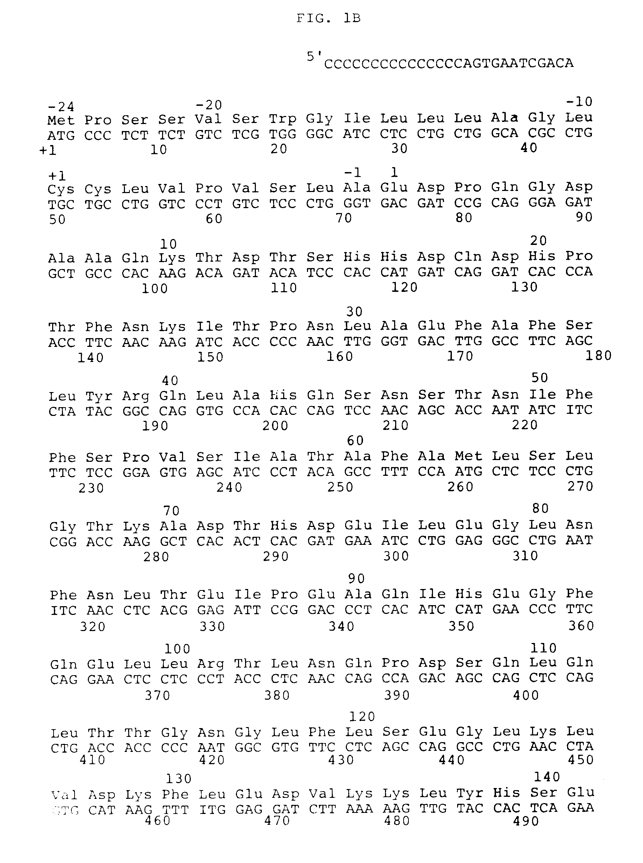

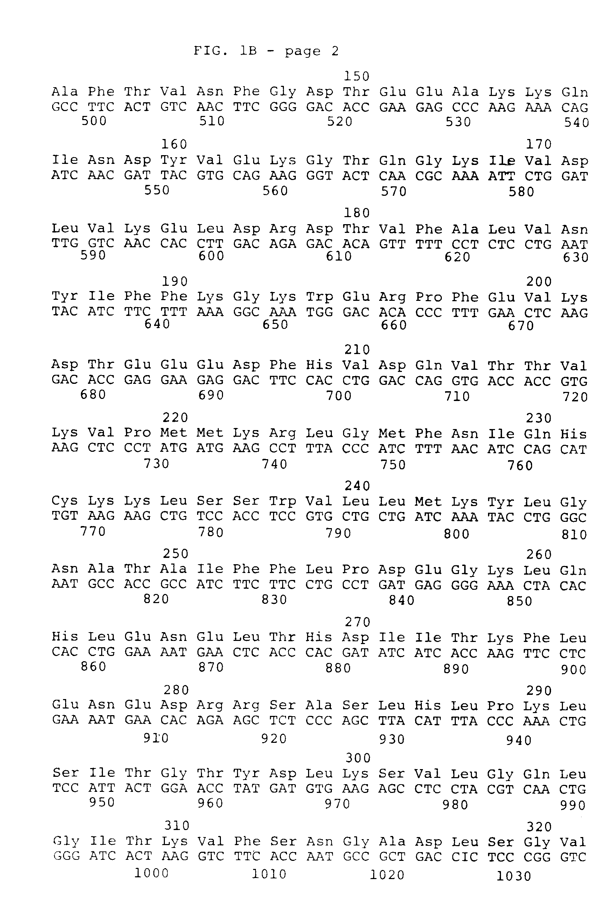

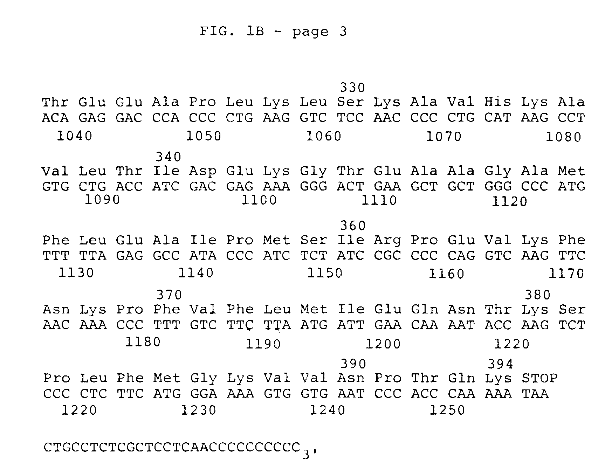

As exemplary of the use of the subject promoters in preparing an active polypeptide composition, as well as being of particular interest for a variety of purposes, a protease inhibitor is described and made. The protease inhibitor has the same or substantially the same amino acid sequence of human alpha-1-antitrypsin and is capable of inhibiting a number of proteolytic enzymes. The human alpha-1-antitrypsin gene appears to reside within a 9.6 kb EcoRI DNA fragment in the human genome. The mature mRNA appears to have about 1400 nucleotides. One human alpha-1-antitrypsin cDNA has the sequence shown in FIG. 1B. The predominant form of human alpha-1-antitrypsin is shown in FIG 1A. Other naturally-occurring forms (polymorphisms) are known.

The sequencing of chromosomal DNA coding for alpha-1-antitrypsin has been described by Kurachi et al., Proc. Natl. Acad. Sci. U.S.A., 78, 6826-6830 (1981) and by Chandra et al., Biochem. Biophys. Res. Comm., 103, 751-758 (1981), the disclosures of which are incorporated herein by reference. A primate gene for alpha-1-antitrypsin may be obtained by DNA cloning methods described by Chandra et al., ibid. The gene coding for the predominant form of human alpha-1-antitrypsin, isolated from a human cDNA library by using the baboon sequence as a DNA hybridization probe is shown in FIG. 1A.

The human alpha-1-antitrypsin has a BamHI restriction site which allows the cutting of the gene with the removal of information for a single glutamic acid from the mature protein. Various schemes can be employed for introducing the human alpha-1-antitrypsin gene adjacent the glycolytic promoter to be under the regulation of the promoter. Where the promoter does not have a convenient restriction site near the f-met codon, the glycolytic gene may be cleaved and chewed back to the promoter with Bal31. A linker may then be introduced downstream from the promoter to provide a convenient cohesive end or flush end for joining to the human alpha-1-antitrypsin gene. The linker can also provide one or more codons for amino acids at the N-terminus of the alpha-1-antitrypsin gene, which may be the same or different from the naturally occurring amino acids.

The gene for human alpha-1-antitrypsin may then be inserted into the extrachromosomal element downstream from the glycolytic promoter, where an f-met codon is provided for initiation of expression of the human alpha-1-antitrypsin.

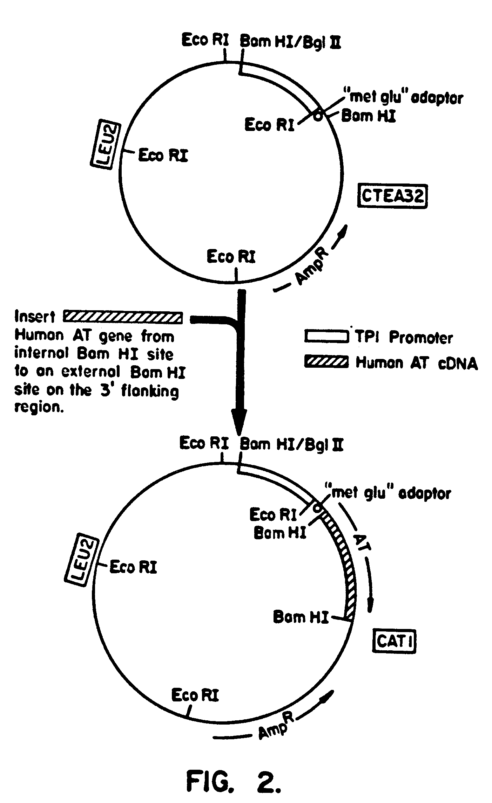

For example, the cDNA coding for alpha-1-antitrypsin (hereinafter "AT") may then be inserted into an expression vector, such as CTEA32 (FIG. 2), which contains the yeast promoter for triose phosphate isomerase (TPI) inserted at the BamHI site of the shuttle plasmid, CV13 [Broach J.R., Strathern J.N., Hicks J.B., Gene, 8:121-133 (1979)]. A synthetic DNA adaptor was ligated into the TPI promoter after the TPI structural sequences were removed by BAL31 digestion from the KpnI restriction site within the TPI coding region. (Alber et al, J. Molec. Applied Genet., 1, 419-434 (1982)). This adaptor contained an ATG codon for translation initiation, followed by the sequence GAGGATCC. The GAG codon specifies a glutamic acid residue, which is the first amino acid of the naturally-occurring human AT. The GGATCC portion of the adaptor is a cutting site for BamHI endonuclease and allows for the splicing of the remainder of human AT DNA sequence into this vector.

The BamHI site of CTEA32 was constructed to be "in frame" with the rest of the AT structural gene, thereby allowing for the expression of the polypeptide when a BamHI fragment from the cloned cDNA is appropriately inserted into CTEA32. The plasmid consisting of CTEA32 plus the AT gene is called CAT1 (FIG. 2).

This DNA construct containing the gene for human AT located downstream to a yeast triose phosphate isomerase (TPI) promoter fragment was transformed into yeast strains, N501-B and GK100. Transformation into yeast is described by Beggs, Nature, 275, 104-109 (1978). Screening of the transformed yeast strains by immunological assays (competition assays and ELISA assays, using antibodies against alpha-1-antitrypsin) confirmed the presence of large amounts of human AT in yeast made from the plasmid CAT1. The "wild-type" yeast strain, N501-1B (described by Kawasaki et al., Biochem. Biophys. Res. Comm., 108, 1107-1112 (1982)), when transformed with CAT1, produced 1.8 mg alpha-1-antitrypsin per gram of soluble protein (or 0.18% alpha-1-antitrypsin), when grown at 30° on a synthetic minimal medium (modified Wickerham's medium) with 6% glucose. A mutant yeast strain, GK100, when transformed with CAT1, produced 10-15 mg alpha-1-antitrypsin per gram soluble protein (or 1-1.5% alpha-1-antitrypsin) under the same growth conditions. Strains N501-1B and GK100 each carry a defective LEU2 gene which allows for the selective maintenance on minimal and leucine-less media of CV13 and CV13-derived plasmids (such as CAT1) which each contain a functional LEU2 gene. When grown on minimal media with only CV13 as a control, N501-1B and GK100 produce no detectable AT. Thus, AT may be specifically produced by the CAT1 plasmid.

Since GK100 produces significantly more AT than N501-1B, it is preferred. However, the present invention is not limited to AT production by GK100. It may be desirable to utilize mutations in GK100 which lead to hyperproduction of AT.

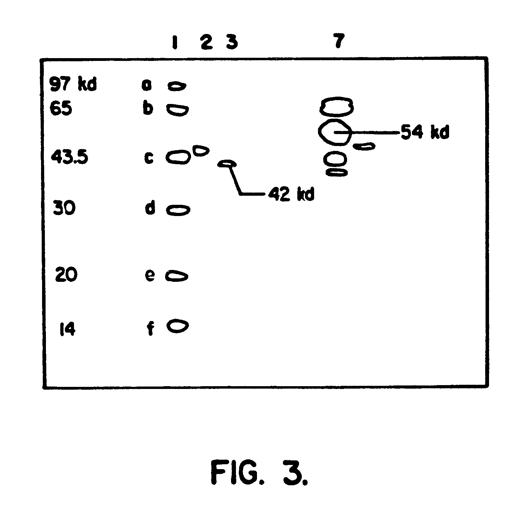

An immuno-adsorption column, made according to the method of Cuatrecasas, P. J. Biol. Chem., 245, 3059 (1970), was prepared by covalently attaching affinity-purified goat antibodies to human AT to CNBr-activated Sepharose. Disrupted GK100 yeast cells were extracted with 3 volumes of phosphate buffered saline pH 7.2 containing 0.5M NaCl, and the extracts were applied to the column. Yeast produced human AT (0.5-1.0 mg) was eluted from the column with 3M NaSCN. After the material was dialyzed to remove salt it was analyzed by electrophoresis on a polyacrylamide gel in the presence of sodium dodecyl sulfate, the results of which are shown in FIG. 3. Based on the relative migration of the protein in the gel, the approximate molecular weight of the human alpha-1-antitrypsin made in yeast is 42,000-43,000 daltons. Naturally occurring human AT has a molecular weight of approximately 54,000 daltons, having a carbohydrate composition of approximately 16% by weight, as shown by Hodges et al, J. Biol. Chem., 254, 8208-8212 (1979). It therefore appears that the yeast produced AT may be unglycosylated or substantially unglycosylated and may lack carbohydrate portions present in the naturally occurring protein.

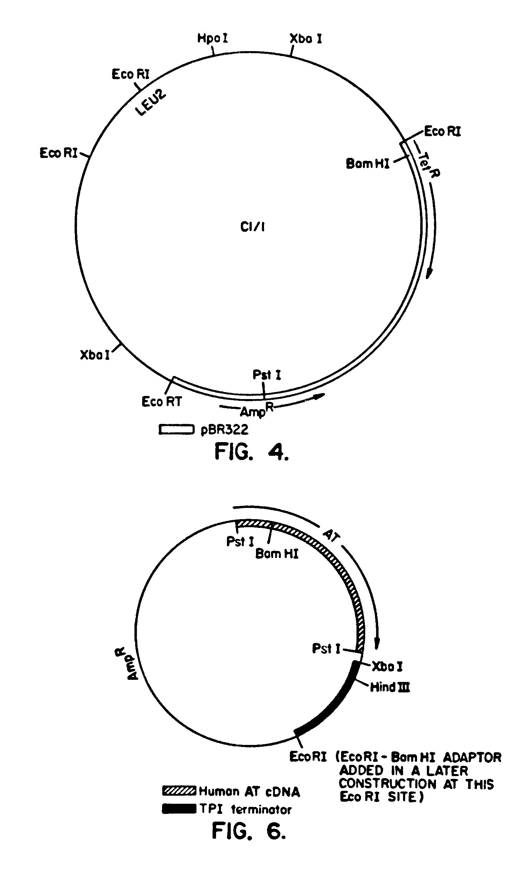

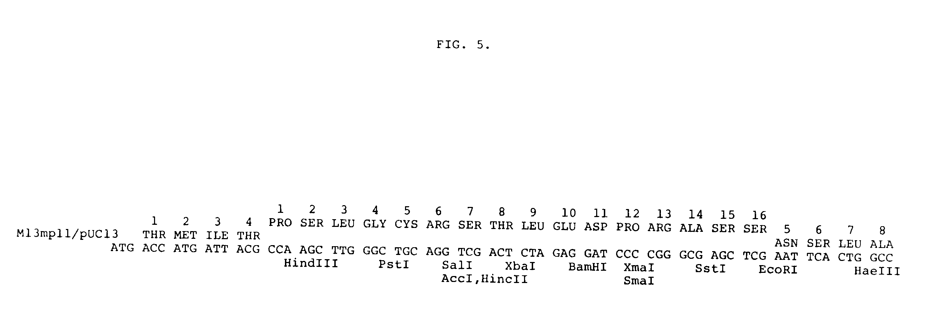

Alternatively, other expression vectors may be constructed which contain a segment coding for alpha-1-antitrypsin. Such expression vectors may be constructed by methods known to those of ordinary skill in the art using available DNA constructs. A preferred vector is plasmid C1/1, which is more stable than CV13 and CV13 derived vectors, such as CAT1. G1/1 was constructed from plasmid, pJDB248 (Beggs, J., Nature, 275, 104-109 (1978)). The pMB9 sequences were removed from pJDB248 by partial digestion with Eco RI and were replaced by pBR322 DNA which was cut with Eco RI. The restriction map of C1/1 is given in FIG. 4. The C1/1 plasmid contains the entire 2-micron DNA from yeast (S.cervisiae), with a pBR322 insertion at an EcoRI site. It also contains the LEU2 gene. Thus, the yeast TPI promotor with the adaptor may be inserted into the single BamHI site in the Tc$^R$ gene of C1/1. Then the AT sequence, attached to a transcription terminator fragment from the yeast TPI gene, may be inserted into the BamHI site downstream from the TPI promotor. The resulting plasmid, HAT4, may then be transformed into N501-1B and GK100 in a manner as described above.

The sequence of the first ten amino acids in the yeast-produced AT may be confirmed by amino acid sequence analysis as identical to the first ten amino acids of the naturally occurring human AT:

$$\text{H}_2\text{N-Glu}\overset{1}{-}\text{Asp}\overset{2}{-}\text{Pro}\overset{3}{-}\text{Gln}\overset{4}{-}\text{Gly}\overset{5}{-}\text{Asp}\overset{6}{-}\text{Ala}\overset{7}{-}\text{Ala}\overset{8}{-}\text{Gln}\overset{9}{-}\text{Lys}\overset{10}{-}$$

The yeast-produced AT does not contain the initiation methionine which is specified by the ATG start codon. Therefore, the yeast cell processes off the methionine to produce the amino acid sequence of natural human AT.

The polypeptides produced according to the present invention having AT activity may be useful for treatment of a genetic AT deficiency and other diseased states related to inadequate levels of AT. Thus, conditions such as emphysema and other lung disorders related to progressive digestion of lung sacs may be treated, such as, chronic obstructive pulmonary disease or adult respiratory distress syndrome. Non-genetically related emphysema may also be treated, such as, emphysema resulting from heavy smoking. Conditions not necessarily confined to the lungs may also be treated, such as, cystic fibrosis and arthritis. For a review of AT deficiency, see Gadek, J.E., and R. Crystal, "Alpha-1-Antitrypsin Deficiency", The Metabolic Basis of Inherited Disease, J.B. Stanbury, J.B. Wyngaarden, D.S. Fredrickson, McGraw-Hill, N.Y. pp. 1450-67 (1982).

The alpha-1-antitrypsin can be used as an antigen for production of polyclonal and monoclonal antibodies to human alpha-1-antitrypsin, for introduction into a host having a deficiency of alpha-1-antitrypsin, or for modulating proteolytic activity in a mammalian host. In particular, the alpha-1-antitrypsin can be administered to humans to replace alpha-1-antitrypsin which has been inactivated (oxidized) by tobacco and other smoke.

The polypeptides according to the present invention may be admixed with conventional pharmaceutical carriers. Preferably, the polypeptides are to be administered intravenously or by inhalation. While the effective dosages may vary according to the severity of the condition and weight of the subject, dosages in the range of 0.5-10.0 gm/week of a polypeptide introduced intravenously may, in many cases, be effective. Lower dosages may be effective if the method of administration is by inhalation. Oral administration may also be effective provided the AT is protected in capsules or coated carriers from premature degradation in the digestive tract.

The following examples set forth specific embodiments according to the present invention, but the invention is not intended to be limited thereto.

EXAMPLE 1

Strains. Isogenic strains carrying mutations in PGI1, PGK1, GPM1, PYK1, and GCR1 where obtained by ethyl methane sulfonate (EMS) mutagenesis of S. cerevisiae (S. c.) X2180-1A (MATa SUC2 CUP1 gal2, from the Berkeley Yeast Stock Center). 35,000 independent colonies were grown on YEP-3% glycerol-2% ethanol and were screened by replica plating for the inability to grow on YEP-4% dextrose (Table 1).

Identification of specific lesions was made by complementation tests with known glycolysis mutants (Ciriacy and Breitenbach, J.Bacteriol, 139:152-60 (1979)), while at least 15 additional complementation groups were found by intercrossing mutant strains. Enzyme assays (Clifton et al. Genetics, 88:1-11 (1980)) confirmed the glycolytic defects in pgi1, pgk1, gpm1, pyk1, and gcr1 mutants.

A LEU2 mutant was also derived from S.cerevisiae X2180-1A by EMS treatment and was crossed to X2180-1B (an isogenic MATα strain) to produce N501-1B (MATα leu2 SUC2 CUP1 gal2). Cycloheximide (cyh2) and canavanine (can1) resistances were then selected as spontaneous mutations in N501-1B. The glycolysis mutants were crossed to N501-1B to produce a series of isogenic leu2 strains each defective in a single glycolytic function or in GCR1.

A tpi1 mutant, S. cerevisiae GLU77 was crossed to N551-1A (MATa leu2 SUC2 CUP1 gal2); strains derived from this mating were crossed twice to N501-1B to produce a tpi1 leu2 strain, N587-2D, which was similar in genetic background to the other glycolysis mutants.

Mutations in three glucose phosphorylating enzymes produce a strain which is unable to grow on dextrose as the sole carbon source and which is resistant to catabolite repression by 2-deoxyglucose and glucosamine. N517-6C (hxk1 hxk2 glk1 leu2 can1-100 cyh2 ade2-1) was derived from a hxk1 hxk2 glk1 strain, D308.3, by screening for glucosamine- resistant spore colonies. Defects in glucose kinasing activities were confirmed by assay.

## TABLE 1

Complementation Groups of glu⁻ Derivatives of X2180-1A

| Gene | No. of Mutants |
|------|----------------|
| PYK1 | 14 |
| PDC1 | 9 |
| GCR1 | 4 |
| PGI1 | 3 |
| GPM1 | 3 |
| PGK1 | 1 |
| TPI1 | 0 |
| FDP | 0 |
| (LEU2) | (1) |

| | |
|------|----|
| I | 11 |
| II | 10 |
| III | 3 |
| IV | 5 |
| V | 1 |
| VI | 1 |
| VII | 2 |
| VIII | 3 |
| IX | 2 |
| X | 3 |
| XI | 2 |
| XII | 1 |
| XIII | 1 |
| XIV | 5 |
| XV | 1 |

60 other mutations not in
the complementation groups

27 sterile glu⁻ strains

35,000 colonies screened (EMS mutagenized for 50% kill)

The homothallic diploid strain, S. c. AB320 was the source of the yeast DNA pool (Nasmyth and Reed, Proc. Nat. Acad. Sci., 77:2119-2123 (1980)) and was used as a control in some experiments.

The triose phosphate isomerase gene (including the upstream sequence having the regulatory signals) is as follows:

The pyruvate kinase gene upstream sequence having the regulatory signals is as follows:

```
        10         20         30         40         50         60
GAATTCACCA TGATAGCTAC GTAAATGTGT TCCGCACCGT CACAAACTGT TTTCTACTGT
CTTAAGTCGT ACTATCGATG CATTTACACA AGGCGTGGCA GTGTTTCACA AAAGATGACA

        70         80         90        100        110        120
TCTTTCTTCT TTCGTTCATT CAGTTCAGTT GACTCAGTGC TTTGTTCAAT GGATCTTAGC
AGAAAGAAGA AAGCAAGTAA GTCAAGTCAA CTCACTCACG AAACAAGTTA CCTAGAATCG

       130        140        150        160        170        180
TAAAATGCAT ATTTTTTCTC TTGCTAAATG AATGCTTGTG ATGTCTTCCA AGTGATTTCC
ATTTTACGTA TAAAAAAGAG AACCATTTAC TTACGAACAC TACAGAAGGT TCACTAAAGG

       190        200        210        220        230        240
TTTCCTTCCC ATATGATGCT AGCTACCTTT ACTGTCTTCC TAAAAAAAAA AAAAGGCTCG
AAAGGAAGGG TATACTACGA TCGATGGAAA TGACAGAAGG ATTTTTTTTT TTTTCCGAGC

       250        260        270        280        290        300
CCATCAAAAC GATATTCGTT GGCTTTTTTT TCTGAATTAT AAATACTCTT TGGTAACTTT
GGTAGTTTTG CTATAAGCAA CCGAAAAAAA AGACTTAATA TTTATGAGAA ACCATTGAAA

       310        320        330        340        350        360
TCATTTCCAA GAACCTCTTT TTTCCAGTTA TATCATCGTC CCCTTTCAAA GTTATTCTCT
AGTAAAGGTT CTTGGAGAAA AAAGGTCAAT ATAGTACCAG GGGAAAGTTT CAATAAGAGA

       370        380        390        400        410        420
ACTCTTTTTC ATATTCATTC TTTTTCATCC TTTCGTTTTT TATTCTTAAC TTGTTTATTA
TGAGAAAAAG TATAAGTAAG AAAAAGTAGG AAAGCAAAAA ATAAGAATTG AACAAATAAT

       430        440        450        460        470        480
TTCTCTCTTG TTTCTATTTA CAAGACACCA ATCAAAACAA ATAAAACATC ATCACAATGT
AAGAGAGAAC AAAGATAAAT GTTCTGTGGT TAGTTTTGTT TATTTTGTAG TAGTGTTACA

       490        500        510        520        530        540
CTAGATTACA AACATTGACC TCATTAAACG TTGTTGCTGG TTCTGACTTC AGAAGAACCT
GATCTAATCT TTGTAACTGG AGTAATTTGC AACAACGACC AAGACTGAAC TCTTCTTGGA

       550        560        570        580        590        600
CCATCATTGG TACCATCGGT TCAAAGACCA ACAACCCAGA AACCTTGGTT GCTTTGACAA
GGTAGTAACC ATGGTAGCCA AGTTTCTGGT TGTTGGGTCT TTGGAACCAA CGAAACTCTT

       610        620        630        640        650        660
AGGCTGGTTT GAACATTGTT CGTATGAACT TCTCTCACGG TTCTTACGAA TACCACAAGT
TCCGACCAAA CTTGTAACAA GCATACTTGA AGAGAGTGCC AAGAATGCTT ATGGTGTTCA

       670        680        690        700
CTGTCGTTGA CAACGCCACA AACTCCGAAG AATTGTACCC
GACAGCAACT GTTGCGGTCT TTGAGGCTTC TTAACATGGG
```

Screening of clone bank. The leu2 glycolysis mutants were transformed with a yeast DNA pool inserted into pYE13, a high copy plasmid carrying a selectable LEU2 wild-type gene (Broach et al., Gene, 8:121-133 (1979)). The glycolytic genes were obtained by complementation, involving the simultaneous selection for growth on glucose and leucine prototrophy. A synthetic medium containing yeast nitrogen base, 4% glucose, and the following supplements was used: per liter, 40mg adenine, 20mg arginine, 50mg aspartate, 10mg histidine, 60mg isoleucine, 40mg lysine, 10mg methionine, 60mg phenylalanine, 50mg threonine, 40mg tryptophan, 50mg tyrosine, 20mg uracil, and 60mg valine.

The transformants were purified on leucineless media and were then grown on a non-selective medium (YEPGE) to allow mitotic segregation of the plasmids. Strains which cosegregated the leu2 and glycolysis mutant phenotypes, as determined by replica plating on selective media, were assayed for glycolytic enzyme activities. Yeast DNA preps were made, and the E. coli strain, RR1, was transformed, selecting for ampicillin resistance, to verify the presence of plasmid DNAs in these yeast glycolytic transformants.

Enzyme Assays. The transformed yeast strains were selectively grown on minimal medium with 8% glucose (adenine was added to a final concentration of 50mg/l). The wild-type control, N501-1B, was grown on the same medium plus leucine (100mg/l). The glycolysis mutant strains were grown on YEP-5% glycerol-1% lactate. Overnight cultures were fed fresh media and were aerobically grown at 30° for four hours before harvesting. The cells were washed two times with water and resuspended in 50mM $K_2HPO_4$, 2mM EDTA, 3mM 2-mercaptoethanol (adjusted to pH7.4 with HCl). Extracts were obtained by vortexing the cells with an equal volume of glass beads (.45 mm diam.) at high speed for two minutes. The cell debris was removed by centrifugation in a microfuge for 15 min. at 4° Enzymes were assayed as described by Clifton and Breitenbach, supra. Protein concentrations were determined by the Biuret-TCA method.

EXAMPLE 2

In order to determine the activity of the various glycolytic genes in the transformants, the various enzymes were assayed and the results for the transformants were compared to mutant and wild-type strains. The gcr1 mutant had 5-10% of the wild-type levels of most glycolytic activities (exemplified by PGI, aldolase and enolase) and grows very poorly on glucose media. In contrast, the GCR1 transformants had nearly wild-type levels of enzymes and were virtually identical to wild-type for growth on glucose media. The other glycolysis mutants had less than 5% of the normal levels of their respective enzyme activities. However, when transformed with a complementing high copy plasmid, the specific enzyme activities were substantially elevated above wild-type levels (typically 5-10 fold higher). The following Table 2 indicates the results.

## TABLE 2

### Comparison of Glycolytic Activities in Wild-type, Mutant, and Transformed Strains

| Enzyme | Activities | | | |
|---|---|---|---|---|
| | | | | Ratio: |
| | Wild-type[a] | Mutant[b] | Transformant[c] | Transf/Wt |
| PGI | 2.85 | .0065 | 31.49 (10) | 11.1 |
| TPI | 18.3 | .0000 | 167.8 (10) | 9.2 |
| PGK | 1.99 | .0046 | 17.67 (3) | 8.9 |
| GPM | 0.74 | .0000 | 4.80 (10) | 6.5 |
| PYK | 4.02 | .0057 | 14.77 (10) | 3.7 |
| | Wild-type[a] | gcr1 Mutant[d] | GCR1 Transf[c] | |
| PGI | 2.85 | .2436 | 2.42 (10) | .85 |
| Aldolase | 4.33 | .4415 | 2.96 (10) | .68 |
| Enolase | 0.43 | .0274 | .316 (10) | .74 |

[a] Wild-type is N501-1B.

[b] The respective mutant strains are N543-9D (pgi1 leu2), N587-2D (tpi1 leu2), N548-8A (pkg1 leu2), N583-2C (gpm1 leu2), and N549-3A (pyk1 leu2).

[c] The activities of the transformants are averages for many different isolates. The numbers in parentheses represent the numbers of independent transformants assayed.

[d] The gcr1 leu2 mutant strain is N525-2C.

EXAMPLE 3

In order to demonstrate that the hyperproduction of glycolytic enzymes was specific to the mutational defect complemented by the particular plasmid, assays for ten different glycolytic proteins were conducted on the various transformants. The following Table 3 reports the results for one transformant for each of the six different glycolysis genes which were examined in detail.

TABLE 3

RELATIVE ENZYME ACTIVITIES OF WILD-TYPE AND TRANSFORMED

| Strains | GLYCOLYTIC ENZYMES | | | | | | |
|---|---|---|---|---|---|---|---|
| | GLK | PGI | PFK | FDA | TPI | GLD | PGK |
| N501-1B | 1.00 | 1.00 | 1.00 | 1.00 | 1.00 | 1.00 | 1.0 |
| Transformant GCR-8 | 1.05 | 0.63 | 1.44 | 0.79 | 0.62 | 0.63 | 0.7 |
| Transformant PGI-19 | 0.64 | 5.63 | 1.26 | 0.57 | 0.58 | 0.75 | 0.5 |
| Transformant TPI-10 | 0.99 | 0.77 | 1.35 | 0.99 | 13.85 | 0.87 | 0.6 |
| Transformant PGK-2 | 0.54 | 0.45 | 1.05 | 0.54 | 0.46 | 0.63 | 2.9 |
| Transformant GPM-2 | 0.97 | 0.82 | 1.69 | 1.02 | 1.02 | 0.85 | 0.9 |
| Transformant PYK-1 | 1.02 | 0.83 | 1.09 | 0.89 | 1.22 | 0.84 | 1.2 |

The GCR-8 transformant gave nearly wild-type levels of all ten enzymes, while PGI-19, TPI-10, PGK-2, GPM-2 and PYK-1 transformants overproduced their respective glycolytic proteins, but not other enzymes.

It was noted that the plasmids readily segregated (typically 5-50% segregation in fully grown cultures even under selective pressure of leucine prototrophy), so the assayed cultures probably contain cells with a range of number of plasmids. By complementation in E. coli and/or sequencing, TPI1 and PYK1 have both been shown to be the structural gene.

EXAMPLE 4

Exploitation of the promoter for TPI1 for the production of human alpha-1-antitrypsin was demonstrated as follows. The plasmid CV13 was employed. CV13 can be maintained by selection of yeast with an average of about ten copies per cell. CV13 is comprised of pBR322, the replicon for the 2μ-plasmid and the yeast LEU2 gene. TPI1 promoter fragment was obtained by cutting the TPI1 gene at the unique KpnI site (bases 511 to 518); and the resulting linearized DNA was then treated with Bal31 for four to five minutes in

14

order to remove the TPI1 structural sequences. Linkers, either EcoRI, Hind III or BamHI, were then inserted. The linkers will then cleave with the appropriate restriction enzyme to provide cohesive ends for insertion of human alpha-1-antitrypsin genes. The human alpha-1-antitrypsin gene was digested with BamHI, which cleaves at the 5'-terminus of the coding strand to remove the information for a single glutamic acid codon from the mature protein. Four different constructions were prepared, as set forth in the following Table 4. From this table it is noted that the glutamic acid codon is substituted by the codons for alanine and proline in three of the constructions having the initiator methionine. After ligation of the human alpha-1-antitrypsin construction into the CV13 plasmid, the resulting plasmid was transformed into S. c. N501-1B. The resulting yeast cells were then grown on a minimal synthetic medium.

TABLE 4

| Plasmid | N-terminal amino acid | Orientation in CV13 |
|---------|----------------------|---------------------|
| CAT1 | met glu + hAT* | clockwise |
| C-Tα2 | met ala pro + hAT | counterclockwise |
| C-Tα1 | met ala pro + hAT | clockwise |
| C-TSα2 | met ala pro + hAT, but missing part of TPI promoter | counterclockwise |

*remainder of approximately 400 amino acids of human alpha-1-antitrypsin

Yeast cells containing the human alpha-1-antitrypsin genes were broken open by vortexing with glass beads (0.45mm) at high speed for 2-3 minutes. The extraction buffer contained 50mM $K_2HPO_4$, 2mM EDTA, 2mM 2-mercaptoethanol and 1mM PMSF (pH7.4). Cell debris was removed by centrifugation and the extracts contain 3-4mg/ml protein as determined by Lowry assays.

The presence of human alpha-1-antitrypsin was determined using a RIA, employing tritium-labeled human alpha-1-antitrypsin and antibody directed against the protein. The following Table 5 indicates the results.

## TABLE 5

### Competition assay for alpha-1 antitrypsin

| Plasmid | Tritium Counts | Average Count | α-1-AT [μg] | Total Protein (μg) | %Total Protein |
|---|---|---|---|---|---|
| CAT1 | 46010 52257 | 49133.5 | 0.75 | 420 | .18 |
| C-Tα2 | 12268 13330 | 12799 | 3.35 | 380 | .88 |
| C+Tα1 | 41635 39071 | 40353 | 0.95 | 360 | .26 |
| C-TSα2 | 66490 70038 | 68264 | 0 | 345 | 0 |

| Controls** | Counts |
|---|---|
| 0 μg α-1 | 68440 |
| 0.25 μg α-1 | 65333 |
| 0.5 μg α-1 | 58928 |
| 1.0 μg α-1 | 38468 |
| 2.0 μg α-1 | 19559 |
| 3.0 μg α-1 | 14432 |
| 4.0 μg α-1 | 11155 |
| 5.0 μg α-1 | 9615 |

*Plasmids were grown in yeast strain, N501-1B. 100μl of extracts were assayed.

**Non-radioactive alpha-1 antitypsin mixed with 100μl of yeast extract (330μg protein)

It is evident from the above results that an immunologically active product is obtained, which is capable of competing with naturally occurring human alpha-1-antitrypsin for antibodies to the native protein.

Furthermore, the expression of the alpha-1-antitrypsin gene is regulated by the TPI promoter, for as is seen, where a portion of the TPI promoter is removed, no alpha-1-antitrypsin is produced. In addition, the production of the mammalian protein human alpha-1-antitrypsin has not been optimized in the above study, so that the results indicate a minimum production of product which can be further enhanced. Thus, the TPI promoter is found to be an effective promoter for efficiently producing high yields of expression products of alien DNA.

EXAMPLE 5

Purification Of Alpha-1-Antitrypsin From Yeast GK100 Yeast Extracts

An immuno adsorption column was prepared by covalently attaching affinity-purified antibodies to human alpha-1-antitrypsin to CNBr-activated Sepharose according to the method of Cuatrecasas, J. Biol. Chem., 245, 3059 (1970). Disrupted GK100 cells were extracted with three volumes of phosphate buffered saline pH 7.2 containing 0.5M NaCl and applied to the column. The column was eluted with 3M NaSCN and the recovered material was analyzed by electrophoresis on polyacrylamide gel in the presence of sodium dodecyl sulfate. The results of the electrophoresis are shown in FIG. 3. Track 1 contained a mixture of molecular weight standards: a) phosphorylase B, 97,000 daltons; b) bovine serum albumen (BSA), 65,000 daltons; c) ovalbumin, 43,500 daltons; d) carbonic anhydrase, 30,000 daltons; e) soybean trypsin inhibitor, 20,000 daltons; and f) alpha-lactalbumin, 14,000 daltons. Track 3 contains yeast produced AT purified by immunoadsorption, molecular weight about 42,000 daltons. Track 7 is a sample of naturally occurring AT purchased from Sigma Chemical Company, heavily contaminated by blood proteins. A major component of Track 7 is human alpha-1-antitrypsin, molecular weight 54,000 daltons.

EXAMPLE 6

Activity Of Yeast Produced Alpha-1-Antitrypsin Against Serine Protease Trypsin

As a control, 10 microliters (1 microgram) of a solution of 100 microgram/ml trypsin, 100 microgram (100 microliters) of bovine serum albumin and 100 microliters of 0.05 molar Tris, pH 8.0 buffer containing 1mM benzoylarginioyl-p-nitroanilide were mixed, and the increase in absorbance at 405 nm was measured over time in a spectrophotometer. The absorbance value of this solution was used as a standard for 100% trypsin activity. Three additional samples were run in duplicate, each containing 1 $\mu$l trypsin and, respectively containing 25 $\mu$l AT solution plus 175 $\mu$l buffer, 100 $\mu$l alpha-1-antitrypsin plus 100 $\mu$l buffer, and 200 $\mu$l alpha-1-antitrypsin. All samples contain equal concentrations of substrate and bovine serum albumin. The results demonstrated that utilizing 25 microliters of AT, 73% of the trypsin activity remained, with 100 microliters of AT, 41% of trypsin activity remained and with 200 microliters of alpha-1-antitrypsin, 26% of the trypsin activity remained. This demonstrated that by increasing the levels of the yeast made AT the trypsin inhibitory activity also increased.

EXAMPLE 7

Production of Alpha-1-Antitrypsin From Yeast Plasmids With Increased Genetic stability

Increased levels of AT may be obtained by utilizing C1/1, a plasmid which is more genetically stable than CV13. The C1/1 plasmid contains the entire 2-micron DNA from S. cerevisiae and, therefore, can promote its own replication and maintenance in yeast in the absence of selection for a genetic marker. Also, C1/1 plasmid has a single BamHI site located in the Tc$^R$ gene. Transformants carrying C1/1 may be selected in E. coli by ampicillin- or tetracycline-resistance and in yeast by leucine prototrophy. C1/1 contains pBR322 inserted into an EcoRI site of 2-micron DNA and carries the LEU2 gene described by J. Beggs, Nature, 275, 104-109 (1978).

The yeast TPI promoter (from CTEA32) with the synthetic DNA adaptor (described above) was inserted as a Bg1 II - BamHI fragment (about 900 base pairs) into the BamHI site of C1/1. This insertion created a single BamHI site into which the human AT gene could be spliced for expression in yeast. As in the CAT1 plasmid, when the AT gene (FIG. 1A) is inserted, the resultant plasmid would have an ATG initiation codon followed by a GAG (glutamic acid codon) to allow the production of mature human AT protein sequence in yeast.

About 700 base pairs of the 3' flanking region of the yeast TPI gene was added after the human AT sequence to assist in transcription termination. The "termination" fragments are sequences from the XbaI to EcoRI sites in the plasmidpTPIC10 (T. Alber and G. Kawasaki, J. Molec. Applied Genet., 1, 419-434 (1982)).

The yeast termination sequences were attached to the human AT gene by using the vector, pUC13, which has multiple cloning sites into which the terminator and AT DNA's can be separately inserted. The pUC13 plasmid was constructed as described in Vieira, J., and Messing, J., Gene, 19, 259-268 (1982) for vectors, pUC8 and pUC9. The pUC13 plasmid contained the multiple restriction site, depicted in FIG. 5, at the start of the lac Z gene. To connect the human AT gene to the TPI transcription terminator, the AT cDNA

17

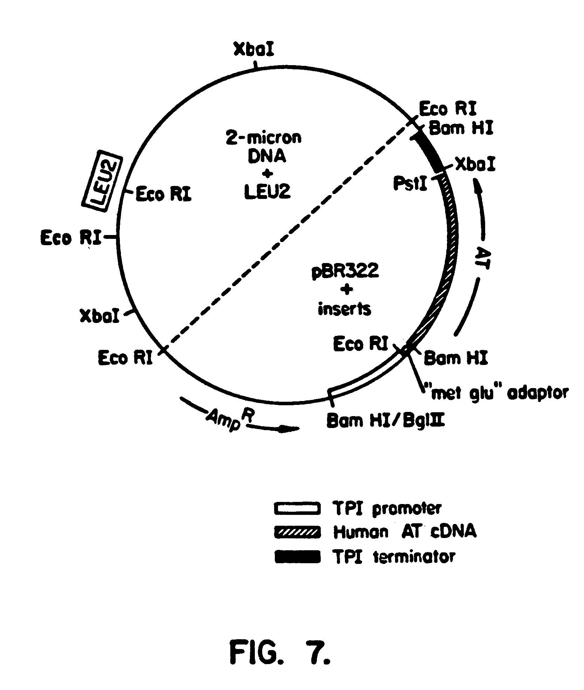

clone (FIG. 1) was inserted as a Pst I fragment into pUC13 at the single Pst I site. The AT gene was followed by an Xba I site and Eco RI site in the multiple cloning sequence. Between these Xba I and Eco RI sites of pUC13 was inserted the yeast TPI terminator as a 700 base pair Xba I-Eco RI fragment from pTPIC10. The resulting plasmid, pUCα1+FG1, contained a human AT gene with a yeast transcription terminator (See FIG. 6). An Eco RI-Bam HI synthetic DNA adaptor was then added to the Eco RI site of the plasmid, in order to create a Bam HI site on the 5' end of the yeast terminator. By using this adaptor, the human AT-yeast terminator sequence could be removed by cutting with Bam HI to liberate a fragment of approximately 2100 base pairs. This BamHI fragment was inserted into the C1/1 plasmid containing the TPI promoter with BamHI adaptor. The resulting plasmid, HAT4, has the TPI promoter, ATGGAGGATCC adapter, human AT gene (from the BamHI site), and TPI terminator inserted into C1/1. The topology of HAT4 is depicted in FIG. 7.

HAT4 was transformed into N501-1B and GK100. On minimal media with 6% glucose, 2-3% of the yeast soluble protein was alpha-1-antitrypsin at a cell density of nearly 3g per liter (wet weight). Because HAT4 contained C1/1, this plasmid was maintainable in a variety of rich media, including YEPD (1% yeast extract, 2% peptone, and 2% glucose). On rich media 2-3% AT was still produced but at a higher cell density of 10-20g per liter (wet weight). The HAT4 plasmid was maintained without selection in N501-1B for over 30 divisions on rich media with greater than 70% of the cells containing the plasmid. In GK100 better than 95% of the cells had HAT4 after 30 divisions on rich media. The advantages of using HAT4 over CAT1 were 1) greater plasmid stability, 2) higher levels of AT as a percentage of total protein, 3) much greater yields of cells per liter as a result of using rich media, and 4) cheaper costs of rich media compared to synthetic (leucine-less) media. The mutant yeast strain GK100 has been placed on deposit in the American Type Culture Collection, Rockville, Maryland, ATCC No. 20669.

It is evident from the above results that yeast promoters can be efficiently used for the production of foreign proteins by regulating the expression of alien DNA in yeast. The promoters are found to be strong promoters, so as to provide for a high degree of expression. Furthermore, it would appear that the messengers are sufficiently stable as to allow for a significant degree of translation into the desired expression product. Furthermore, by employing the glycolytic promoters and appropriate nutrient media, the expression of the alien DNA can be modulated. In this way, production of the alien DNA can be turned on and off. Thus, the subject invention provides a method for using yeast as efficient host in the production of foreign proteins, where the production may be modulated. In addition, by using the glycolytic regulation gene, one can turn on and off a plurality of glycolytic promoters.

**Claims**

1. An extrachromosomal element capable of replication in yeast, having a marker for selection in a yeast host and containing a yeast promoter which regulates the transcription of triose phosphate isomerase, the promoter being present in the following sequence:

```
GAATCCATCAATAGATACGTCCTCAGGACCGTGCTACCCAAATGCACTCATTGTCAGCCA
-610        -600        -590        -580        -570        -560

GACCTAACTACATAGTGTTTAAGATTACCGATATTTAACTTACTTACAATAATGCCATTT
-550        -540        -530        -520        -510        -500

TTTTGAGTTATAATAATCCTACGTTAGTGTGCAGCCGGCATTTAAACTCTCACCGACCTTAAT
-490        -480

ACATTCAGACACTTCTGACGCTATCACCCTACTTATTCCCTTCCAGATTATATCTAGGAA
-470        -460        -450        -440        -430        -420

CCCATCACGCTTGGTGGAACATTACCCGTTCTAAGACTTTTCAGCCTTCCTCTATTCATCTT
-410        -400        -390        -380        -370        -360

ACACTTCGACACCCCTTTTCTCGCATCCAGTTTTTAATCTTCAGTGGCCATGTCGCAGATTCT
-350        -340        -330        -320        -310        -300

CCGAAATTAATTAAAGCAATCACACAATTCTCTCGGATACCACCTCCGTTCAAACTGACA
-290        -280        -270        -260        -250        -240

GGTGGTTTCTTACGCATGCTAATGCAAAGCAGCCTATATACCCTTTGGCCTCCGCTGCTGTA
-230        -220        -210        -200        -190        -180

ACAGGCAATATAAAGGGCCAGCCATAATTTACCGACTTTAGTCAACTTGCAACATTTACTATT
-170        -160        -150        -140        -130        -120

TTCCCTTCTTACGTAAATATTTTTCTTTTTTAATTCTAAATCAATCTTTTTCAATTTTTTC
-110        -100        -90         -80         -70         -60

TTTGTATTCTTTTCTTGCTTAAATCTATAACTACAAAAAAACACATACATAAACTAAAAAT
-50         -40         -30         -20         -10         +1 1
```

```
ALA ASN THR PHE PHE VAL GLY GLY ASN PHE LYS LEU ASN GLY SER LYS GLN SER ILE LYS
GCCTACAACTTTCTTTGTCGGTCGTAACTTTAAAATTAAACCGTTCCAAACAATCCATTAA
        10          20          30          40          50          60

GLU ILE VAL GLU ARG LEU ASN THR ALA SER ILE PRO GLU ASN VAL GLU VAL VAL ILE CYS
CGAAATTGTTCAAAGATTGAACACTGCTTCTATCCCAGAAAATGTCGAACTTGTTATCTC
        70          80          90          100         110         120

PRO PRO ALA THR TYR LEU ASP TYR SER VAL SER LEU VAL LYS LYS PRO GLN VAL VAL THR VAL
TCCTCCAGCTACCTACTTAGACTACTCTGTCTCTTTGGTTAAGAAGCCACAACTCACTGT
        130         140         150         160         170         180

GLY ALA GLN ASN ALA TYR LEU LYS ALA SER GLY ALA PHE THR GLY GLU ASN SER VAL ASP
CGGTGCTCAAAACGCCTACTTGAAGGCTTCTGGTGCTTTCACCGGTGAAAACTCCGTTGA
        190         200         210         220         230         240

GLN ILE LYS ASP VAL GLY ALA LYS TRP VAL ILE LEU GLY HIS SER GLU ARG ARG SER TYR
CCAAATCAAGGATGTTGGTGCTAAGTCCGGTTATTTTGGGTCACTCCGAAAGAACATCTTA
        250         260         270         280         290         300

PHE HIS GLU ASP ASP LYS PHE ILE ALA ASP LYS THR LYS PHE ALA LEU GLY GLN GLY VAL
CTTCCACCAAGATGACAAGTTCATTGCTGACAAGACCAAGTTCGCTTTACCTCAAGGTGT
        310         320         330         340         350         360

GLY VAL ILE LEU CYS ILE GLY GLU THR LEU GLU GLU LYS LYS ALA GLY LYS THR LEU ASP
CGGTGTCATCTTGTGTATCGGTGAAACTTTGGAAGAAAAGAAGGCCGGTAAGACTTTGGA
        370         380         390         400         410         420

VAL VAL GLU ARG GLN LEU ASN ALA VAL LEU GLU GLU VAL LYS ASP TRP THR ASN VAL VAL
TGTTGTTCAAAGACAATTGAACGCTGTCTTGGAAGAAGTTAAGGACTGGACTAACGTCGT
        430         440         450         460         470         480

VAL ALA TYR GLU PRO VAL TRP ALA ILE GLY THR GLY LEU ALA ALA THR PRO GLU ASP ALA
TGTCGCTTACGAACCAGTCTGGGCCATTGGTACCGGTTTGGCTGCTACTCCAGAAGATGC
        490         500         510         520         530         540

GLN ASP ILE HIS ALA SER ILE ARG LYS PHE LEU ALA SER LYS LEU GLY ASP LYS ALA ALA
TCAAGATATTCACGCTTCCATCAGAAAGTTCTTGGCTTCCAAGTTGGGTCACAAGGCTGC
        550         560         570         580         590         600

SER GLU LEU ARG ILE LEU TYR GLY GLY SER ALA ASN GLY SER ASN ALA VAL THR PHE LYS
CAGCGAATTGAGAATCTTATACGGTGGTTCCGCTAACGGTAGCAACGCCGTTACCTTCAA
        610         620         630         640         650         660

ASP LYS ALA ASP VAL ASP GLY PHE LEU VAL GLY GLY ALA SER LEU LYS PRO GLU PHE VAL
GGACAAGGCTGATGTCGATGGTTTCTTGGTCGGTGGTGCTTCTTTGAAGCCAGAATTTGT
        670         680         690         700         710         720

ASP ILE ILE ASN SER ARG ASN ***
TGATATCATCAACTCTAGAAACTAAGATTAATATAATTATATAAAAATATTATCTTCTTT
        730         740         747 +1      +10         +20         +30
```

```
TCTTTATATCTAGTGTTATGTAAAATAAAATTCATCACTACGCAAAGCTTTTTTATATTGT
+40         +50         +60         +70         +80         +90

TTCTTTTTCATTCTGAGCCACTTAAAATTTCGTGAATCTTCTTGTAAGGCACGGTACATTT
+100        +110        +120        +130        +140        +150

ACAAGTGATACAACAAAAAGCAAGGCCGCTTTTTCTAATAAAAACAACAAAAGCATTTAAC
+160        +170        +180        +190        +200        +210

AATTCAACACCTCTATATCAACACGAACA
+220        +230        +240
```

or pyruvate kinase, the promoter being present in the following sequence:

```
         10        20        30        40        50        60
CAATTCACCA TCATACCTAC GTAAATGTGT TCCGCACCGT CACAAACTGT TTTCTACTGT
CTTAAGTCGT ACTATCGATG CATTTACACA AGGCGTGGCA GTGTTTCACA AAACATGACA

         70        80        90       100       110       120
TCTTTCTTCT TTCGTTCATT CAGTTCAGTT GACTCACTGC TTTGTTCAAT GGATCTTAGG
ACAAACAAGA AAGCAAGTAA GTCAAGTCAA CTGAGTGACG AAACAAGTTA CCTAGAATCG

        130       140       150       160       170       180
TAAAATGCAT ATTTTTTCTC TTGGTAAATG AATGCTTGTG ATGTCTTCCA AGTGATTTCC
ATTTTACGTA TAAAAAAGAG AACCATTTAC TTACGAACAC TACAGAAGGT TCACTAAAGG

        190       200       210       220       230       240
TTTCCTTCCC ATATGATGCT AGGTACCTTT AGTGTCTTCC TAAAAAAAAA AAAAGGCTCG
AAAGGAACGG TATACTACGA TCCATGGAAA TCACAGAAGG ATTTTTTTTT TTTTCCGAGC

        250       260       270       280       290       300
CCATCAAAAC GATATTCGTT GGCTTTTTTT TCTGAATTAT AAATACTCTT TGGTAACTTT
GGTAGTTTTG CTATAAGCAA CCGAAAAAAA ACACTTAATA TTTATGAGAA ACCATTGAAA

        310       320       330       340       350       360
TCATTTCCAA GAACCTCTTT TTTCCAGTTA TATCATGGTC CCCTTTCAAA GTTATTCTCT
AGTAAAGGTT CTTGGAGAAA AAAGGTCAAT ATAGTACCAG GGGAAAGTTT CAATAAGAGA

        370       380       390       400       410       420
ACTCTTTTTC ATATTCATTC TTTTTCATCC TTTCGTTTTT TATTCTTAAC TTGTTTATTA
TGAGAAAAAG TATAAGTAAG AAAAAGTAGG AAAGCAAAAA ATAAGAATTG AACAAATAAT

        430       440       450       460       470       480
TTCTCTCTTG TTTCTATTTA CAAGACACCA ATCAAAACAA ATAAAACATC ATCACAATGT
AAGAGAGAAC AAAGATAAAT GTTCTGTGGT TAGTTTTGTT TATTTTGTAG TAGTGTTACA

        490       500       510       520       530       540
CTAGATTACA AACATTGACC TCATTAAACG TTGTTGCTGG TTCTGACTTG AGAAGAACCT
GATCTAATGT TTGTAACTGG AGTAATTTGC AACAACGACC AAGACTGAAC TCTTCTTGGA

        550       560       570       580       590       600
CCATCATTGG TACCATGGGT TCAAAGACCA ACAACCCAGA AACCTTGGTT GCTTTGACAA
GGTAGTAACC ATGGTAGCCA AGTTTCTGGT TGTTGGGTCT TTGGAACCAA CGAAACTCTT

        610       620       630       640       650       660
AGGCTGGTTT GAACATTGTT CGTATGAACT TCTCTCACGG TTCTTACGAA TACCACAAGT
TCCGACCAAA CTTGTAACAA GCATACTTGA AGAGAGTGCC AAGAATGCTT ATGGTGTTCA

        670       680       690       700
CTGTCGTTGA CAACGCCAGA AACTCCGAAG AATTGTACCC
GACAGCAACT GTTGCGGTCT TTGAGGCTTC TTAACATGGG
```

in either case the yeast promoter being followed downstream by a DNA sequence coding for a protein having protease inhibition activity under the regulation of the promoter.

2. An extrachromosomal element as claimed in claim 1, wherein the promoter is followed by a gene expressing protease inhibitor activity having the amino acid sequence of human alpha-1-antitrypsin.

3. An extrachromosomal element as claimed in claim 1, wherein the DNA sequence is a human alpha-1-antitrypsin cDNA.

4. An extrachromosomal element as claimed in claim 1, which includes a triose phosphate isomerase terminator.

5. A method for preparing a protein having protease inhibition activity which comprises: introducing into a yeast host an extrachromosomal element as claimed in any one of claims 1 to 4 and growing the yeast host in an appropriate medium and isolating the protein expressed.

6. A yeast cell containing an extrachromosomal element as claimed in any one of claims 1 to 4.

**7.** A yeast cell containing at least a portion of an extrachromosomal element according to any of claims 1 to 4 integrated into the genome of the yeast cell, wherein the portion includes at least the promoter and the DNA sequence.

**8.** Plasmid HAT4 characterised by the restriction map shown in Figure 7.

**9.** Plasmid CATI, characterised by the restriction map shown in Figure 2.

**10.** Plasmid pUCα1, characterized by the restriction map shown in Figure 6.

## Patentansprüche

**1.** Zur Replikation in Hefe fähiges extrachromosomales Element mit einem Marker für die Selektion in einem Hefewirt, das einen Hefepromotor enthält, der die Transkription der Triosephosphatisomerase reguliert, wobei der Promotor in der folgenden Sequenz vorhanden ist:

```
GAATCCATCAATAGATACGTCCTCAGGACCGTGCTACCCAAATGCACTCATTCTCAGCCA
-450      -440      -430      -420      -410      -400
CACCTAACTACATAGTGTTTAACATTACCGATATTTAACTTACTTAGAATAATGCCATTT
-590      -580      -570      -560      -550      -540
TTTTGAGTTATAATAATCCTACGTTAGTCTCAGCCGGCATTTAAACTCTCAGCACCTTAAT
-530      -520      -510      -500      -490      -480
ACATTCAGACACTTCTCACCGTATCACCCTACTTATTCCCTTCGAGATTATATCTAGCAA
-470      -460      -450      -440      -430      -420
CCCATCAGCTTCGTCCAACGATTACCCGTTCTAAGACTTTTCAGCTTCCTCTATTCATCTT
-410      -400      -390      -380      -370      -360
ACACTTCCACACCCCTTTTCTCGGCATCCAGTTTTTAATCTTCAGTCGCATGTCGACATTCT
-350      -340      -330      -320      -310      -300
CCGAAATTAATTAAAGCCAATCACACAATTCTCTCGGATACCACCTCCGTTCAAACTCACA
-290      -280      -270      -260      -250      -240
GGTCGTTTCGTTACGCATGCTAATGCAAACCAGCCCTATATACCTTTGCCTCCGCCTTGCTGTA
-130      -120      -210      -200      -190      -180
ACAGGCAATATAAAGCGCCAGCATAATTTACCCAGTTTAGTCGAACTTGCAACATTTACTATT
-170      -160      -150      -140      -130      -120
TTCCCTTCTTACGTAAATATTTTTTCTTTTTAATTCTAAATCAATCTTTTTCAATTTTTTTC
-110      -100      -90       -80       -70       -60
TTTGTATTCTTTTCTTGCTTAAATCTATAACTACAAAAAACACATACATAAACTAAAAAT
-50       -40       -30       -20       -10       -1 1
```

```
 ALA ARG THR PHE PHE VAL GLY GLY ASN PHE LYS LEU ASN GLY SER LYS GLN ASP ILE LYS
CGCTACAACTTTCTTTCGTCGGTCGTAACTTTAAATTAAACCGTTCCAAACAATCCATTAA
        10          20          30          40          50          60
 GLU ILE VAL GLU ARG LEU ASN THR ALA SER ILE PRO GLU ASN VAL GLU VAL VAL VAL ILE CYS
CGAAATTGTTCAAAGATTCAACACTGCTTCTATCCCAGAAAATGTCGAAGTTGTTATCTG
        70          80          90          100         110         120
 PRO PRO ALA THR TYR LEU ASP TYR SER VAL SER LEU VAL LYS LYS PRO GLN VAL THR VAL
TCCTCCAGCTACCTACTTAGACTACTCTGTCTCTTTGGTTAAGAAGCCACAAGTCACTGT
        130         140         150         160         170         180
 GLY ALA GLN ASN ALA TYR LEU LYS ALA SER ALA PHE THR GLY GLU ASN SER VAL ASP
CGGTGCTCAAAACGCCTACTTGAAGGCTTCTGCTTTCACCCGTGAAAACTCCGTTGA
        190         200         210         220         230         240
 GLN ILE LYS ASP VAL GLY ALA LYS TRP VAL ILE LEU GLY HIS SER GLU ARG ARG SER TYR
CCAAATCAAGGATGTTGGTGCTAAGTGCCTTATTTTGGGTCACTCCGAAAGAACATCTTA
        250         260         270         280         290         300
 PHE HIS GLU ASP ASP LYS PHE ILE ALA ASP LYS THR LYS PHE ALA LEU GLY GLN GLY VAL
CTTCCACGAAGATCACAAGTTCATTGCTGCACAACACCAAGTTCGCTTTAGCTCAAGGTCT
        310         320         330         340         350         360
 GLY VAL ILE LEU CYS ILE GLY GLU THR LEU GLU GLU LYS LYS ALA GLY LYS THR LEU ASP
CGGTGTCATCTTGTGTATCGGTGAAACTTTGGAAGAAAAGAAGGCCGGTAAGACTTTGGCA
        370         380         390         400         410         420
 VAL VAL GLU ARG GLN LEU ASN ALA VAL LEU GLU GLU VAL LYS ASP TRP THR ASN VAL VAL
TGTTGTTGAAAGACAATTGAACGCTGTCTTGGAAGAAGTTAAGGACTGGACTAACGTCGT
        430         440         450         460         470         480
 VAL ALA TYR GLU PRO VAL TRP ALA ILE GLY THR GLY LEU ALA ALA THR PRO GLU ASP ALA
TGTCGCTTACGAACCAGTCTGGGCCATTCGTACCGGTTTGGCTGCTACTCCAGAACAACATGCC
        490         500         510         520         530         540
 GLN ASP ILE HIS ALA SER ILE ARG LYS PHE LEU ALA SER LYS LEU GLY ILE ALA ALA
TCAAGATATTCACGCTTCCATCAGAAAGTTCTTGGCTTCCAAGTTGGGTCACAACGCTGC
        550         560         570         580         590         600
 SER GLU LEU ARG ILE LEU TYR GLY GLY SER ALA ASN GLY SER ASN ALA VAL THR PHE LYS
CAGCGAATTCAGAATCTTATACGGTGGTTCCCCTAACGGTACCAACGCCCTTACCTTCAA
        610         620         630         640         650         660
 ASP LYS ALA ASP VAL ASP GLY PHE LEU VAL GLY GLY ALA SER LEU LYS PRO GLU PHE VAL
GGACAAGGCCGATGTCGATGGTTTTCTTGGTCGTCGTCCTGTCCTTCTTTGAAGCCAGAATTTGT
        670         680         690         700         710         720
 ASP ILE ILE ASN SER ARG ASN ***
TGATATCATCAACTCTAGAAACTAAGATTAATATAATTATATAAAAATATTATCTTCTTT
        730         740     747 +1        +10       +20       +30
TCTTTATATCTAGTGTTATGTAAAAATAAATTGATGCACTACCGCAAAGCTTTTTTTATATTGT
    +40          +50          +60          +70          +80          +90
TTCTTTTTCATTCTGCAGCCACTTAAATTTCGTCAATGTTCTTGTAAGGCACGGTAGATTT
    +100         +110         +120         +130         +140         +150
ACAAGTGATACAACAAAAAGCAAGCCGCTTTTTCTAATAAAAAGAACAAAAAGCATTTAAC
    +160         +170         +180         +190         +200         +210
AATTCAACACCTCTATATCAACAGAACA
    +220         +230         +240
```

oder die der Pyruvatkinase, wobei der Promotor in der folgenden Sequenz vorhanden ist:

EP 0 103 409 B1

```
          10         20         30         40         50         60
     CAATTCACCA TCATACCTAC GTAAATGTGT TCCCCACCGT CACAAACTGT TTTCTACTGT
     GTTAAGTCGT ACTATCGATG CATTTACACA AGCCGTCCCA GTGTTTCACA AAACATCACA

          70         80         90        100        110        120
     TCTTTCTTCT TTCGTTCATT CAGTTCAGTT GACTGACTGC TTTGTTCAAT CCATCTTACC
     AGAAAGAAGA AAGCAAGTAA GTCAAGTCAA CTCACTCACG AAACAAGTTA GGTACAATGG

         130        140        150        160        170        180
     TAAAATGCAT ATTTTTTCTC TTGGTAAATG AATGCCTTGTG ATGTCTTCCA AGTCATTTCC
     ATTTTACGTA TAAAAAAGAG AACCATTTAC TTACCAACAC TACACAAGGT TCAGTAAAGG

         190        200        210        220        230        240
     TTTCCTTCCC ATATGATGCT AGCTACCTTT AGTGTCTTCC TAAAAAGAAA AAAAGCCTCG
     AAAGGAACCG TATACTACGA TCCATCGAAA TCACACAAGG ATTTTTTTTT TTTTCCGAGC

         250        260        270        280        290        300
     CCATCAAAAC GATATTCGTT GGGTTTTTTTT TCTCAATTAT AAATACTCTT TGGTAACTTT
     GGTAGTTTTG CTATAAGCAA CCCAAAAAAA ACACTTAATA TTTATGAGAA ACCATTGAAA

         310        320        330        340        350        360
     TCATTTCCAA CAACCTCTTT TTTCCACTTA TATCATCGTC CCCTTTCAAA GTTATTCTCT
     AGTAAACGTT CTTGGACAAA AAAGGTCAAT ATAGTACCAG GGGAAAGTTT CAATAAGACA

         370        380        390        400        410        420
     ACTCTTTTTC ATATTCATTC TTTTTCATCC TTTCGTTTTT TATTCTTAAC TTGTTTATTA
     TGACAAAAAG TATAAGTAAC AAAAAGTAGG AAACCAAAAA ATAAGAATTG AACAAATAAT

         430        440        450        460        470        480
     TTCTCTCTTC TTTCTATTTA CAAGACACCA ATCAAAACAA ATAAAACATC ATCACAATGT
     AAGAGAGAAG AAAGATAAAT GTTCTGTGGT TAGTTTTGTT TATTTGTAG TAGTGTTACA

         490        500        510        520        530        540
     CTAGATTACA AACATTGACC TCATTAAACG TTGTTGCTGG TTCTCACTTG ACAACAACCT
     GATCTAATGT TTCTAACTGG AGTAATTTGC AACAACGACC AAGACTGAAG TCTTCTTGGA

         550        560        570        580        590        600
     CCATCATTGG TACCATCGGT TCAAAGACCA ACAACCCAGA AACCTTCGTT GCTTTGACAA
     GGTAGTAACC ATGGTAGCCA AGTTTCTGGT TGTTGGGTCT TTGGAACCAA CGAAACTCTT

         610        620        630        640        650        660
     AGCCTCGTTT CAACATTGTT CCTATCAACT TCTCTCACCG TTCTTACCAA TACCACAAGT
     TCCGACCAAA CTTGTAACAA GCATACTTCA AGAGAGTGCC AACAATGCTT ATGGTGTTCA

         670        680        690        700
     CTGTCGTTCA CAACCCCACA AACTCCCAAG AATTGTACCC
     GACACCAACT GTTGCGGTCT TTCAGCCTTC TTAACATGCG
```

wobei dem Hefepromotor in jedem Fall stromabwärts eine DNA-Sequenz folgt, die für ein Protein mit Proteaseinhibitionsaktivität kodiert und von dem Promotor reguliert wird.

2. Extrachromosomales Element nach Anspruch 1, wobei dem Promotor ein Gen folgt, das eine Proteaseinhibitoraktivität exprimiert und die Aminosäuresequenz von humanem $\alpha$-1-Antitrypsin hat.

3. Extrachromosomales Element nach Anspruch 1, wobei die DNA-Sequenz eine cDNA für humanes $\alpha$-1-Antitrypsin ist.

4. Extrachromosonales Element nach Anspruch 1, das einen Triosephosphatisomerase-Terminator umfaßt.

5. Verfahren zum Herstellen eines Proteins mit Proteaseinhibitionsaktivität, umfassend: das Einführen eines extrachromosomalen Elementes nach einem der Ansprüche 1 bis 4 in einen Hefewirt und Wachsenlassen des Hefewirtes in einem entsprechenden Medium und Isolieren des exprimierten Proteins.

6. Hefezelle, enthaltend ein extrachromosomales Element nach einem der Ansprüche 1 bis 4.

22

**7.** Hefezelle, enthaltend in das Genom der Hefezelle integriert mindestens einen Teil eines extrachromosomalen Elementes nach einem der Ansprüche 1 bis 4,wobei der Teil mindestens den Promotor und die DNA-Sequenz umfaßt.

**8.** Plasmid HAT4, gekennzeichnet durch die in Figur 7 gezeigte Restriktionskarte.

**9.** Plasmid CATI, gekennzeichnet durch die in Figur 2 gezeigte Restriktionskarte.

**10.** Plasmid pUCα1, gekennzeichnet durch die in Figur 6 gezeigte Restriktionskarte.

**Revendications**

**1.** Elément extrachromosomique apte à la réplication dans une levure, possédant un marqueur pour la sélection dans un hôte consistant en une levure et contenant un promoteur de levure qui régule la transcription de la triose-phosphate-isomérase, le promoteur étant présent dans la séquence suivante :

23

ou la pyruvate-kinase, le promoteur étant présent dans la séquence suivante :

```
        10         20         30         40         50         60
CAATTCACCA TCATACCTAC GTAAATGTGT TCCGCACCGT CACAAACTGT TTTCTACTGT
CTTAAGTCGT ACTATCCATC CATTTACACA AGCCGTGGCA GTGTTTCACA AAACATGACA

        70         80         90        100        110        120
TCTTTCTTCT TTCCTTCATT CAGTTCAGTT GACTCAGTGC TTTGTTCAAT CCATCTTACC
ACAAACAAGA AAGCAAGTAA GTCAATTCAA CTCACTCACG AAACAAGTTA CCTACAATCG

       130        140        150        160        170        180
TAAAATGCAT ATTTTTTCTC TTGCTAAATG AATCCTTCTG ATGTCTTCCA AGTCATTTCC
ATTTTACGTA TAAAAAACAG AACCATTTAC TTACCAACAC TACAGAAGGT TCAGTAAAGG

       190        200        210        220        230        240
TTTCCTTCCC ATATCATCCT AGCTACCTTT ACTCTCTTCC TAAAAAAAAA AAAACCCTCG
AAACCAACCG TATACTACCA TCCATCCAAA TCACACAAGG ATTTTTTTTT TTTTCCGAGC

       250        260        270        280        290        300
CCATCAAAAC GATATTCCTT GGCTTTTTTT TCTCAATTAT AAATACTCTT TCCTAACTTT
GGTAGTTTTG CTATAAGCAA CCGAAAAAAA ACACTTAATA TTTATGAGAA ACCATTGAAA

       310        320        330        340        350        360
TCATTCCAA GAACCTCTTT TTTCCAGTTA TATCATCGTC CCCTTTCAAA GTTATTCTCT
ACTAAACGTT CTTGGACAAA AAAGGTCAAT ATAGTACCAG GGGAAAGTTT CAATAAGAGA

       370        380        390        400        410        420
ACTCTTTTTC ATATTCATTC TTTTTCATCC TTTCGTTTTT TATTCTTAAC TTGTTTATTA
TGACAAAAAG TATAAGTAAC AAAAAGTAGG AAAGCAAAAA ATAAGAATTG AACAAATAAT

       430        440        450        460        470        480
TTCTCTCTTG TTTCTATTTA CAACACACCA ATCAAAACAA ATAAAACATC ATCACAATGT
AAGAGAGAAC AAAGATAAAT GTTCTGTGGT TAGTTTTGTT TATTTTGTAG TAGTGTTACA

       490        500        510        520        530        540
CTAGATTACA AACATTCACC TCATTAAACG TTGTTCCTCG TTCTGACTTG ACAAGAACCT
GATCTAATGT TTGTAAGTGG ACTAATTTGC AACAAGGAGC AAGACTGAAC TGTTCTTGGA

       550        560        570        580        590        600
CCATCATTGG TACCATCGGT TCAAAGACCA ACAACCCAGA AACCTTGGTT GGTTTGACAA
GGTAGTAACC ATGGTAGCCA AGTTTCTGGT TGTTGGGTCT TTGGAACCAA CCAAACTGTT

       610        620        630        640        650        660
ACCGTCGTTT CAACATTCTT GGTATGAACT TCTCTCACGG TTCTTACCAA TACCACAAGT
TGGGAGCAAA GTTGTAAGAA GGATAGTTGA AGAGAGTGCC AAGAATGGTT ATGGTGTTCA

       670        680        690        700
CTGTGGTTCA GAACGGGACA AAGTGGGAAG AATTGTACGG
GACACCAAGT GTTGGGGTCT TTGACGGTTG TTAAGATGGG
```

le promoteur de levure étant suivi dans chaque cas, en aval, par une séquence d'ADN codant pour une protéine ayant une activité d'inhibition de protéases sous la régulation du promoteur.

2. Elément extrachromosomique suivant la revendication 1, dans lequel le promoteur est suivi par un gène exprimant une activité d'inhibiteur de protéases, ayant la séquence d'amino-acides de l'alpha-1-antitrypsine humaine.

3. Elément extrachromosomique suivant la revendication 1, dans lequel la séquence d'ADN est un ADNc d'alpha-1-antitrypsine humaine.

4. Elément extrachromosomique suivant la revendication 1, qui comprend un terminateur de triose-phosphate-isomérase.

5. Procédé de préparation d'une protéine ayant une activité d'inhibition des protéases, qui consiste : à introduire dans un hôte consistant en une levure un élément extrachromosomique suivant l'une quelconque des revendications 1 à 4, à cultiver la levure-hôte dans un milieu approprié et à isoler la protéine exprimée.

**6.** Cellule de levure contenant un élément extrachromosomique suivant l'une quelconque des revendications 1 à 4.

**7.** Cellule de levure contenant au moins une portion d'un élément extrachromosomique suivant l'une quelconque des revendications 1 à 4 intégrée dans le génome de la cellule de levure, dans laquelle la portion comprend au moins le promoteur et la séquence d'ADN.

**8.** Plasmide HAT4 caractérisé par la carte de restriction représentée sur la figure 7.

**9.** Plasmide CAT1, caractérisé par la carte de restriction représentée sur la figure 2.

**10.** Plasmide pUCα1, caractérisé par la carte de restriction représentée sur la figure 6.

FIG. 1A

```
                                              5'  GGGGGGGGGGGGGGG CA CCA CCA CTG ACC
                                                                10            20

                        -24                    -20                                    -10
                        Met Pro Ser Ser Val Ser Trp Gly Ile Leu Leu Leu Ala Gly Leu
TGG GAC AGT GAA TCG ACA ATG CCG TCT TCT GTC TCG TGG GGC ATC CTC CTG CTG GCA GGC CTG
 30          40          50          60          70          80          90

                                          -1   +1                                10
Cys Cys Leu Val Pro Val Ser Leu Ala Glu Asp Pro Gln Gly Asp Ala Ala Gln Lys Thr Asp
TGC TGC CTG GTC CCT GTC TCC CTG GCT GAG GAT CCC CAG GGA GAT GCT GCC CAG AAG ACA GAT
         100         110         120         130         140         150

                              20                                        30
Thr Ser His His Asp Gln Asp His Pro Thr Phe Asn Lys Ile Thr Pro Asn Leu Ala Glu Phe
ACA TCC CAC CAT GAT CAG GAT CAC CCA ACC TTC AAC AAG ATC ACC CCC AAC CTG GCT GAG TTC
     160         170         180         190         200         210

                          40                                        50
Ala Phe Ser Leu Tyr Arg Gln Leu Ala His Gln Ser Asn Ser Thr Asn Ile Phe Phe Ser Pro
GCC TTC AGC CTA TAC CGC CAG CTG GCA CAC CAG TCC AAC AGC ACC AAT ATC TTC TTC TCC CCA
220         230         240         250         260         270         280

                      60                                        70
Val Ser Ile Ala Thr Ala Phe Ala Met Leu Ser Leu Gly Thr Lys Ala Asp Thr His Asp Glu
GTG AGC ATC GCT ACA GCC TTT GCA ATG CTC TCC CTG GGG ACC AAG GCT GAC ACT CAC GAT GAA
         290         300         310         320         330         340

                  80                                        90
Ile Leu Glu Gly Leu Asn Phe Asn Leu Thr Glu Ile Pro Glu Ala Gln Ile His Glu Gly Phe
ATC CTG GAG GGC CTG AAT TTC AAC CTC ACG GAG ATT CCG GAG GCT CAG ATC CAT GAA GGC TTC
         350         360         370         380         390         400

              100                                        110
Gln Glu Leu Leu Arg Thr Leu Asn Gln Pro Asp Ser Gln Leu Gln Leu Thr Thr Gly Asn Gly
CAG GAA CTC CTC CGT ACC CTC AAC CAG CCA GAC AGC CAG CTC CAG CTG ACC ACC GGC AAT GGC
 410         420         430         440         450         460         470

          120                                        130
Leu Phe Leu Ser Glu Gly Leu Lys Leu Val Asp Lys Phe Leu Glu Asp Val Lys Lys Leu Tyr
CTG TTC CTC AGC GAG GGC CTG AAG CTA GTG GAT AAG TTT TTG GAG GAT GTT AAA AAG TTG TAC
             480         490         500         510         520         530

          140                                        150
His Ser Glu Ala Phe Thr Val Asn Phe Gly Asp Thr Glu Glu Ala Lys Lys Gln Ile Asn Asp
CAC TCA GAA GCC TTC ACT GTC AAC TTC GGG GAC ACC GAA GAG GCC AAG AAA CAG ATC AAC GAT
         540         550         560         570         580         590

160                                        170                                    180
Tyr Val Glu Lys Gly Thr Gln Gly Lys Ile Val Asp Leu Val Lys Glu Leu Asp Arg Asp Thr
TAC GTG GAG AAG GGT ACT CAA GGG AAA ATT GTG GAT TTG GTC AAG GAG CTT GAC AGA GAC ACA
     600         610         620         630         640         650

                          190                                        200
Val Phe Ala Leu Val Asn Tyr Ile Phe Phe Lys Gly Lys Trp Glu Arg Pro Phe Glu Val Lys
GTT TTT GCT CTG GTG AAT TAC ATC TTC TTT AAA GGC AAA TGG GAG AGA CCC TTT GAA GTC AAG
660         670         680         690         700         710         720

                      210                                        220
Asp Thr Glu Glu Glu Asp Phe His Val Asp Gln Val Thr Thr Val Lys Val Pro Met Met Lys
GAC ACC GAG GAA GAG GAC TTC CAC GTG GAC CAG GTG ACC ACC GTG AAG GTG CCT ATG ATG AAG
         730         740         750         760         770         780

                  230                                        240
Arg Leu Gly Met Phe Asn Ile Gln His Cys Lys Lys Leu Ser Ser Trp Val Leu Leu Met Lys
CGT TTA GGC ATG TTT AAC ATC CAG CAC TGT AAG AAG CTG TCC AGC TGG GTG CTG CTG ATG AAA
     790         800         810         820         830         840
```

27

FIG. 1A - page 2

```
                              250                                      260
Tyr Leu Gly Asn Ala Thr Ala Ile Phe Phe Leu Pro Asp Glu Gly Lys Leu Gln His Leu Glu
TAC CTG GGC AAT GCC ACC GCC ATC TTC TTC CTG CCT GAT GAG GGG AAA CTA CAG CAC CTG GAA
850             860         870         880         890         900             910


                        270                                      280
Asn Glu Leu Thr His Asp Ile Ile Thr Lys Phe Leu Glu Asn Glu Asp Arg Arg Ser Ala Ser
AAT GAA CTC ACC CAC GAT ATC ATC ACC AAG TTC CTG GAA AAT GAA GAC AGA AGG TCT GCC AGC
            920         930         940         950         960         970


                  290                                      300
Leu His Leu Pro Lys Leu Ser Ile Thr Gly Thr Tyr Asp Leu Lys Ser Val Leu Gly Gln Leu
TTA CAT TTA CCC AAA CTG TCC ATT ACT GGA ACC TAT GAT CTG AAG AGC GTC CTG GGT CAA CTG
      980         990         1000        1010        1020        1030


            310                                      320
Gly Ile Thr Lys Val Phe Ser Asn Gly Ala Asp Leu Ser Gly Val Thr Glu Glu Ala Pro Leu
GGC ATC ACT AAG GTC TTC AGC AAT GGG GCT GAC CTC TCC GGG GTC ACA GAG GAG GCA CCC CTG
1040        1050        1060        1070        1080        1090            1100


      330                                      340                          350
Lys Leu Ser Lys Ala Val His Lys Ala Val Leu Thr Ile Asp Glu Lys Gly Thr Glu Ala Ala
AAG CTC TCC AAG GCC GTG CAT AAG GCT GTG CTG ACC ATC GAC GAG AAA GGG ACT GAA GCT GCT
            1110        1120        1130        1140        1150        1160


                                            360                     370
Gly Ala Met Phe Leu Glu Ala Ile Pro Met Ser Ile Pro Pro Glu Val Lys Phe Asn Lys Pro
GGG GCC ATG TTT TTA GAG GCC ATA CCC ATG TCT ATC CCC CCC GAG GTC AAG TTC AAC AAA CCC
            1170        1180        1190        1200        1210        1220


                                            380                             390
Phe Val Phe Leu Met Ile Glu Gln Asn Thr Lys Ser Pro Leu Phe Met Gly Lys Val Val Asn
TTT GTC TTC TTA ATG ATT GAA CAA AAT ACC AAG TCT CCC CTC TTC ATG GGA AAA GTG GTG AAT
1230        1240        1250        1260        1270        1280


            394
Pro Thr Gln Lys STOP
CCC ACC CAA AAA TAA CTG CCT CTC GCT CCT CAA CCC CTC CCC TCC ATC CCT GGC CCC CTC CCT
1290        1300        1310        1320        1330        1340        1350



GGA TGA CAT TAA AGA AGG GTT GAG CTG
      1360            1370



 G AAAAAAAAAAAAAAAA CCCCCCCCCCCCCCCCCCCCCCCCCCCCCCCCCCCCCCCCCCC 3'
1380     1390      1400      1410      1420      1430
```

28

FIG. 1B

5' CCCCCCCCCCCCCCCCAGTGAATCGACA

```
-24                  -20                                              -10
Met Pro Ser Ser Val Ser Trp Gly Ile Leu Leu Leu Ala Gly Leu
ATG CCC TCT TCT GTC TCG TGG GGC ATC CTC CTG CTG GCA CGC CTG
+1           10           20           30           40

+1                                   -1   1
Cys Cys Leu Val Pro Val Ser Leu Ala Glu Asp Pro Gln Gly Asp
TGC TGC CTG GTC CCT GTC TCC CTG GGT GAC GAT CCG CAG GGA GAT
50               60           70           80           90

              10                                        20
Ala Ala Gln Lys Thr Asp Thr Ser His His Asp Cln Asp His Pro
GCT GCC CAC AAG ACA GAT ACA TCC CAC CAT GAT CAG GAT CAC CCA
              100          110          120          130

                             30
Thr Phe Asn Lys Ile Thr Pro Asn Leu Ala Glu Phe Ala Phe Ser
ACC TTC AAC AAG ATC ACC CCC AAC TTG GGT GAC TTG GCC TTC AGC
    140          150          160          170              180

              40                                        50
Leu Tyr Arg Gln Leu Ala His Gln Ser Asn Ser Thr Asn Ile Phe
CTA TAC GGC CAG GTG CCA CAC CAG TCC AAC AGC ACC AAT ATC ITC
              190          200          210          220

                             60
Phe Ser Pro Val Ser Ile Ala Thr Ala Phe Ala Met Leu Ser Leu
TTC TCC GGA GTG AGC ATC CCT ACA GCC TTT CCA ATG CTC TCC CTG
    230          240          250          260          270

              70                                        80
Gly Thr Lys Ala Asp Thr His Asp Glu Ile Leu Glu Gly Leu Asn
CGG ACC AAG GCT CAC ACT CAC GAT GAA ATC CTG GAG GGC CTG AAT
              280          290          300          310

                             90
Phe Asn Leu Thr Glu Ile Pro Glu Ala Gln Ile His Glu Gly Phe
ITC AAC CTC ACG GAG ATT CCG GAC CCT CAC ATC CAT GAA CCC TTC
    320          330          340          350          360

              100                                       110
Gln Glu Leu Leu Arg Thr Leu Asn Gln Pro Asp Ser Gln Leu Gln
CAG GAA CTC CTC CCT ACC CTC AAC CAG CCA GAC AGC CAG CTC CAG
              370          380          390          400

                             120
Leu Thr Thr Gly Asn Gly Leu Phe Leu Ser Glu Gly Leu Lys Leu
CTG ACC ACC CCC AAT GGC GTG TTC CTC AGC CAG GCC CTG AAC CTA
    410          420          430          440          450

              130                                       140
Val Asp Lys Phe Leu Glu Asp Val Lys Lys Leu Tyr His Ser Glu
CTG CAT AAG TTT ITG GAG GAT CTT AAA AAG TTG TAC CAC TCA GAA
              460          470          480          490
```

29

FIG. 1B - page 2

```
                              150
Ala Phe Thr Val Asn Phe Gly Asp Thr Glu Glu Ala Lys Lys Gln
GCC TTC ACT GTC AAC TTC GGG GAC ACC GAA GAG CCC AAG AAA CAG
    500             510         520         530         540

              160                                 170
Ile Asn Asp Tyr Val Glu Lys Gly Thr Gln Gly Lys Ile Val Asp
ATC AAC GAT TAC GTG CAG AAG GGT ACT CAA CGC AAA ATT CTG GAT
            550         560         570         580

                              180
Leu Val Lys Glu Leu Asp Arg Asp Thr Val Phe Ala Leu Val Asn
TTG GTC AAC CAC CTT GAC AGA GAC ACA GTT TTT CCT CTC CTG AAT
    590         600         610         620         630

              190                                 200
Tyr Ile Phe Phe Lys Gly Lys Trp Glu Arg Pro Phe Glu Val Lys
TAC ATC TTC TTT AAA GGC AAA TGG GAC ACA CCC TTT GAA CTC AAG
            640         650         660         670

                              210
Asp Thr Glu Glu Glu Asp Phe His Val Asp Gln Val Thr Thr Val
GAC ACC GAG GAA GAG GAC TTC CAC CTG GAC CAG GTG ACC ACC GTG
    680         690         700         710         720

              220                                 230
Lys Val Pro Met Met Lys Arg Leu Gly Met Phe Asn Ile Gln His
AAG CTC CCT ATG ATG AAG CCT TTA CCC ATC TTT AAC ATC CAG CAT
            730         740         750         760

                              240
Cys Lys Lys Leu Ser Ser Trp Val Leu Leu Met Lys Tyr Leu Gly
TGT AAG AAG CTG TCC ACC TCC GTG CTG CTG ATC AAA TAC CTG GGC
    770         780         790         800         810

              250                                 260
Asn Ala Thr Ala Ile Phe Phe Leu Pro Asp Glu Gly Lys Leu Gln
AAT GCC ACC GCC ATC TTC TTC CTG CCT GAT GAG GGG AAA CTA CAC
            820         830         840         850

                              270
His Leu Glu Asn Glu Leu Thr His Asp Ile Ile Thr Lys Phe Leu
CAC CTG GAA AAT GAA CTC ACC CAC GAT ATC ATC ACC AAG TTC CTC
    860         870         880         890         900

              280                                 290
Glu Asn Glu Asp Arg Arg Ser Ala Ser Leu His Leu Pro Lys Leu
GAA AAT GAA CAC AGA AGC TCT CCC AGC TTA CAT TTA CCC AAA CTG
            910         920         930         940

                              300
Ser Ile Thr Gly Thr Tyr Asp Leu Lys Ser Val Leu Gly Gln Leu
TCC ATT ACT GGA ACC TAT GAT GTG AAG AGC CTC CTA CGT CAA CTG
    950         960         970         980         990

              310                                 320
Gly Ile Thr Lys Val Phe Ser Asn Gly Ala Asp Leu Ser Gly Val
GGG ATC ACT AAG GTC TTC ACC AAT GCC GCT GAC CIC TCC CGG GTC
        1000        1010        1020        1030
```

FIG. 1B - page 3

```
                                    330
Thr Glu Glu Ala Pro Leu Lys Leu Ser Lys Ala Val His Lys Ala
ACA GAG GAC CCA CCC CTG AAG GTC TCC AAC CCC CTG CAT AAG CCT
   1040           1050           1060           1070           1080
                340
Val Leu Thr Ile Asp Glu Lys Gly Thr Glu Ala Ala Gly Ala Met
GTG CTG ACC ATC GAC GAG AAA GGG ACT GAA GCT GCT GGG CCC ATG
       1090               1100           1110           1120

                                    360
Phe Leu Glu Ala Ile Pro Met Ser Ile Arg Pro Glu Val Lys Phe
TTT TTA GAG GCC ATA CCC ATC TCT ATC CGC CCC CAG GTC AAG TTC
   1130           1140           1150           1160           1170
                370                                    380
Asn Lys Pro Phe Val Phe Leu Met Ile Glu Gln Asn Thr Lys Ser
AAC AAA CCC TTT GTC TTC TTA ATG ATT GAA CAA AAT ACC AAG TCT
           1180               1190           1200           1220
                                    390           394
Pro Leu Phe Met Gly Lys Val Val Asn Pro Thr Gln Lys STOP
CCC CTC TTC ATG GGA AAA GTG GTG AAT CCC ACC CAA AAA TAA
   1220           1230           1240           1250
```

CTGCCTCTCGCTCCTCAACCCCCCCCCC$_3$,

31

FIG. 2.

FIG. 3.

FIG. 4.

FIG. 6.

FIG. 5.

```
                                          1    2    3    4    5    6    7    8    9    10   11   12   13   14   15   16
                       1    2    3    4   PRO  SER  LEU  GLY  CYS  ARG  SER  THR  LEU  GLU  ASP  PRO  ARG  ALA  SER  SER              5    6    7    8
M13mp11/pUC13         THR  MET  ILE  THR                                                                                            ASN  SER  LEU  ALA
                      ATG  ACC  ATG  ATT  ACG  CCA  AGC  TTG  GGC  TGC  AGG  TCG  ACT  CTA  GAG  GAT  CCC  CGG  GCG  AGC  TCG  AAT  TCA  CTG  GCC
                                               HindIII         PstI       SalI       XbaI      BamHI  XmaI            SstI      EcoRI          HaeIII
                                                                     AccI,HincII                      SmaI
```

FIG. 7.